# EUROPEAN PATENT SPECIFICATION

(11) **EP 1 343 532 B1**
(45) Date of publication and mention of the grant of the patent: **24.04.2013**
(21) Application number: 01985065.0
(22) Date of filing: 18.12.2001
(51) Int. Cl.: A61K 48/00, A61K 35/00

(54) **LENTIVIRAL VECTOR-MEDIATED GENE TRANSFER AND USES THEREOF**
DURCH LENTIVIRALEN VEKTOR VERMITTELTER GENTRANSFER UND ANWENDUNGSZWECKE
TRANSFERT DE GENE INDUIT PAR UN VECTEUR LENTIVIRAL ET UTILISATION DE CELUI-CI

(30) Priority: 19.12.2000 US 256701 P
(43) Date of publication of application: 17.09.2003
(60) Divisional application: 10177185.5
(73) Proprietor: RESEARCH DEVELOPMENT FOUNDATION, Carson City, Nevada 89703 (US)
(72) Inventor: STOUT, J. Timothy, Portland, OR 97229 (US); APPUKUTTAN, Binoy, Portland, OR 97201 (US)
(74) Representative: Mallalieu, Catherine Louise
(86) International application number: PCT/US2001/049241
(87) International publication number: WO 2002/049677

(56) References cited:
- US-A- 5 969 120
- US-A- 6 004 798
- MURATA T ET AL: "The possibility of gene therapy for the treatment of choroidal neovascularization" OPHTHALMOLOGY, J. B. LIPPINCOTT CO., PHILADELPHIA, PA, US, vol. 107, no. 7, July 2000 (2000-07), pages 1364-1373, XP002975540 ISSN: 0161-6420
- MURATA TOSHINORI ET AL: "Ocular gene therapy: Experimental studies and clinical possibilities" OPHTHALMIC RESEARCH, vol. 29, no. 5, 1997, pages 242-251, XP001120114 ISSN: 0030-3747
- SAKAMOTO T ET AL: "Inhibition of experimental proliferative vitreoretinopathy by retroviral vector-mediated transfer of suicide gene" OPHTHALMOLOGY, J. B. LIPPINCOTT CO., PHILADELPHIA, PA, US, vol. 102, no. 10, October 1995 (1995-10), pages 1417-1424, XP002975541 ISSN: 0161-6420
- MARUTA: "Prospects for tumor suppressor gene therapy: RB as an example" TUMOR SUPPRESSING VIRUSES, GENES AND DRUGS. INNOVATIVE CANCER THERAPY APPROACHES, 2002, pages 97-122, XP002975542

## Description

### BACKGROUND OF THE INVENTION

### Field of the Invention

The present invention relates generally to the field of molecular biology of vectors and gene therapy. More specifically, the present invention relates to using lentiviral vectors in human gene therapy for inherited and proliferative ocular disease.

### Description of the Related Art

One of the most common causes of human blindness is abnormal, intraocular cellular proliferation that often results in a loss of clarity of the visual axis or in a separation of the retina from the retinal pigment epithelium (RPE) due to tractional forces applied directly to the retinal surface. Proliferative retinal detachment, whether it is related to proliferative diabetic disease (PDR), retinopathy of prematurity (ROP), proliferative vitreoretinopathy (PVR), or neovascular age-related macular degeneration (AMD), if left untreated, ultimately results in permanent loss of vision.

The abnormal proliferation of new blood vessels within the eye, ocular neovascularization, is the most common cause of permanent blindness in developed countries. Three diseases are associated with the vast majority of all cases of intraocular neovascularization: diabetes, retinopathy of prematurity and age-related macular degeneration. While these three clinical entities are distinct and affect different groups of patients, they share a final common pathway that involves the uncontrolled division of endothelial cells leading to the formation of new blood vessels that ultimately compromise retinal function. Together, these conditions account for approximately 60% of untreatable blindness in the United States.

Proliferation of vascular endothelial cells within the retina initiates the process of proliferative diabetic retinopathy (PDR). If untreated, these endothelial cells continue to divide and eventually form fibrovascular membranes that extend along the inner surface of the retina or into the vitreous cavity. Contraction of the posterior vitreous surface results in traction at the sites of vitreo-fibrovascular adhesions and ultimately detaches the retina. Approximately 50% of Type 1 diabetics will develop proliferative diabetic retinopathy within 20 years of the diagnosis of diabetes, whereas 10% of patients with Type 2 disease will evidence proliferative diabetic retinopathy within a similar timeframe.

Blood vessels usually develop by one of two processes: vasculogenesis or angiogenesis. During vasculogenesis, a primitive network of capillaries is established during embryogenesis by the maturation of multipotential mesenchymal progenitors. In contrast, angiogenesis refers to a remodeling process involving pre-existing vessels. In angiogenesis, new vascular buds emanate from older, established vessels and invade the surrounding tissue. In the retina, once the normal vascular network is established, the remodeling of this network is largely under the influence of tissue oxygen concentration - hypoxia (oxygen paucity) stimulates angiogenesis. It is this process which results in blindness in millions of diabetics, premature infants or the aged in society.

Intraocular diseases such as age-related macular degeneration, proliferative diabetic retinopathy, retinopathy of prematurity, glaucoma, and proliferative vitreoretinopathy are therefore characterized by abnormal proliferation or other states for which gene therapy may be useful. It has been difficult, however, to perform gene transduction in mammalian cells with any great degree of effectiveness. Additionally, results seen with such traditional vectors as adenoviral vectors, liposomes and dendrimer-based reagents are quite transient. It is also problematic to introduce these vectors into the eye without induction of a strong inflammatory response.

Thus, the prior art is deficient in the lack of means of transducing terminally differentiated or proliferating human cells within or derived from the eye. The present invention fulfills this long-standing need and desire in the art.

### SUMMARY OF THE INVENTION

It is an object of the present invention to develop lentiviral vectors and methods of using these vectors in human gene therapy for inherited and proliferative ocular disease. The usefulness of lentiviral vectors is described for the transduction of human retinal, corneal, vascular endothelial, proliferative vitreoretinopathic and retinal pigment epithelial cells.

In one embodiment of the present invention, the potential of suppressing intraocular cell division by a lentiviral-delivered constitutively active (mutant or variant) retinoblastoma (CA-rb) gene was demonstrated. Human ocular cells were tested *in vitro* and two models of intraocular proliferative disease (proliferative vitreoretinopathy and post-lens extraction posterior capsular opacification) were tested *in vivo.* Significant and long-lived inhibition of cell division *in vitro* was observed in many different cell types. Reduction in the severity of proliferative vitreoretinopathy and post-lens extraction posterior capsular opacification were observed *in vivo.*

In another embodiment of the present invention, it was demonstrated that lentivirus-mediated transfer of genes known to be important in the development and inhibition of new blood vessel growth (angiogenesis) or pre-programmed cell death (apoptosis) could be useful in the treatment of pathologic ocular angiogenesis (e.g., diabetic retinopathy or "wet" age related macular degeneration) or pathologic cell death (e.g., "dry" age related macular degeneration). These genes were placed under the control of one of each of two separate strong promoters known to be active in human retinal, corneal and retinal pigment epithelial cells, and inhibition of corneal neovascularization was demonstrated in rabbit model.

In addition, this vectoring system, when harboring genes known to be deficient in human patients with inherited eye disease, can transfer these genes to human ocular cells. The transfer of these genes by this system forms the basis for useful therapies for these patients with eye diseases.

The present invention is drawn to a method of inhibiting intraocular cellular proliferation in an individual in need of such treatment, such as an individual having an ocular disease. This method comprises the step of: administering to said individual a pharmacologically effective dose of a lentiviral vector comprising a therapeutic gene that inhibits intraocular cellular proliferation.

The present invention is also drawn to a method of inhibiting intraocular neovascularization in an individual having an ocular disease, comprising the step of: administering to said individual a pharmacologically effective dose of a lentiviral vector comprising a therapeutic gene that inhibits intraocular neovascularization.

Other and further aspects, features, and advantages of the present invention will be apparent from the following description of the presently preferred embodiments of the invention. These embodiments are given for the purpose of disclosure.

### BRIEF DESCRIPTION OF THE DRAWINGS

So that the matter in which the above-recited features, advantages and objects of the invention, as well as others which will become clear, are attained and can be understood in detail, more particular descriptions of the invention briefly summarized above may be had by reference to certain embodiments thereof which are illustrated in the appended drawings. These drawings form a part of the specification. It is to be noted, however, that the appended drawings illustrate preferred embodiments of the invention and therefore are not to be considered limiting in their scope.

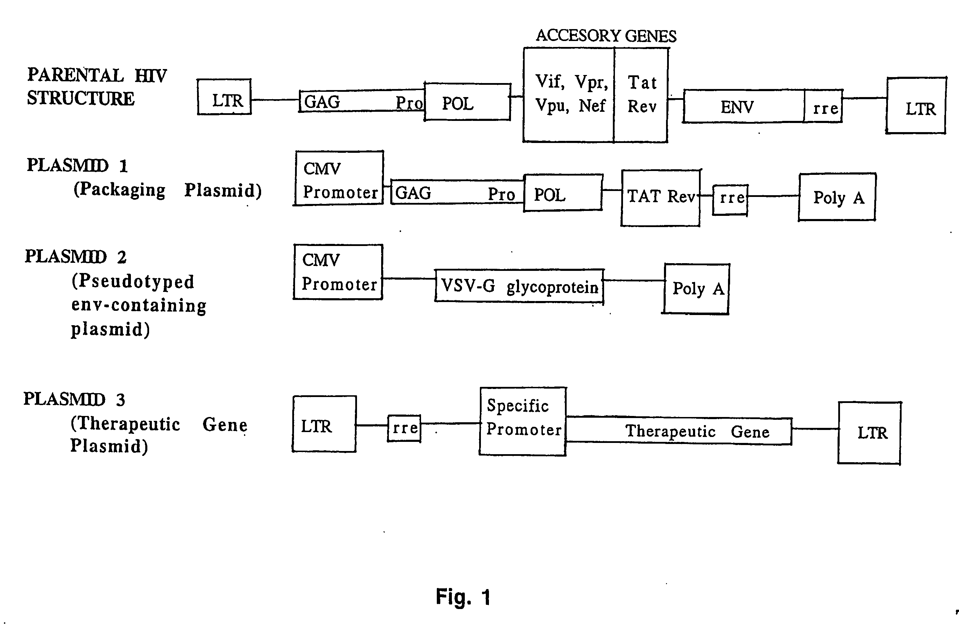

**Figure 1** depicts a vector (provided by Dr. Inder Verma, Salk Institute, San Diego, CA). HIV: human immunodeficiency virus, LTR: long terminal repeat, GAG: HIV GAG gene, POL: HIV reverse transcriptase, ENV: HIV envelope gene, rre: rev-responsive element, CMV: cytomegalovirus, VSV: vesicular stomatitis virus, Poly A: polyadenylation signal, Specific promoter: any transcription-enhancing promoter can be place here so as to modulate spatial, temporal or quantitative aspects of therapeutic gene expression, Therapeutic gene: any gene with therapeutic potential can be placed here - examples include, but are not limited to, a constitutively active retinoblastoma gene, or genes whose deficiency results in disease.

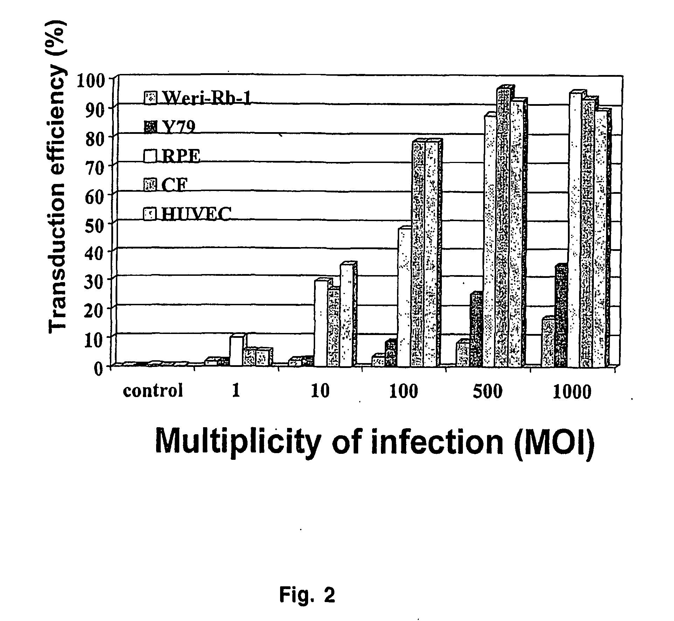

**Figure 2** shows *in vitro* transduction of the following human cell lines: human retinal pigment epithelial cells (RPE), human umbilical vein endothelial cells (HUVEC), Choroidal fibroblasts (CF), human retinoblastoma (retinal-derived) cells (Weri-Rb-1 and Y79). These cell lines were transduced with lentiviral particles containing a marker gene (the enhanced green fluorescent protein gene) and the fraction of cells expressing the marker gene were determined by fluorescent-activated cell sorting. A dose-response is noted as more cells are transduced with greater numbers of lentiviral particles (multiplicity of infection - MOI)

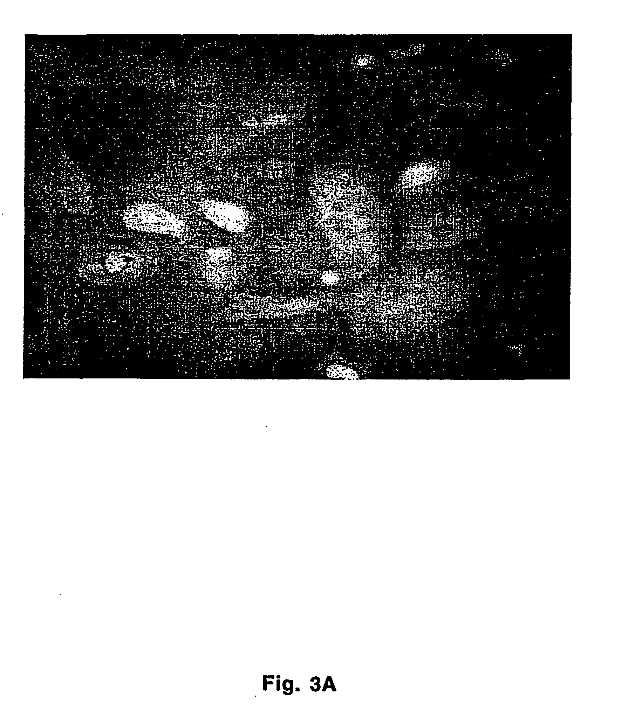

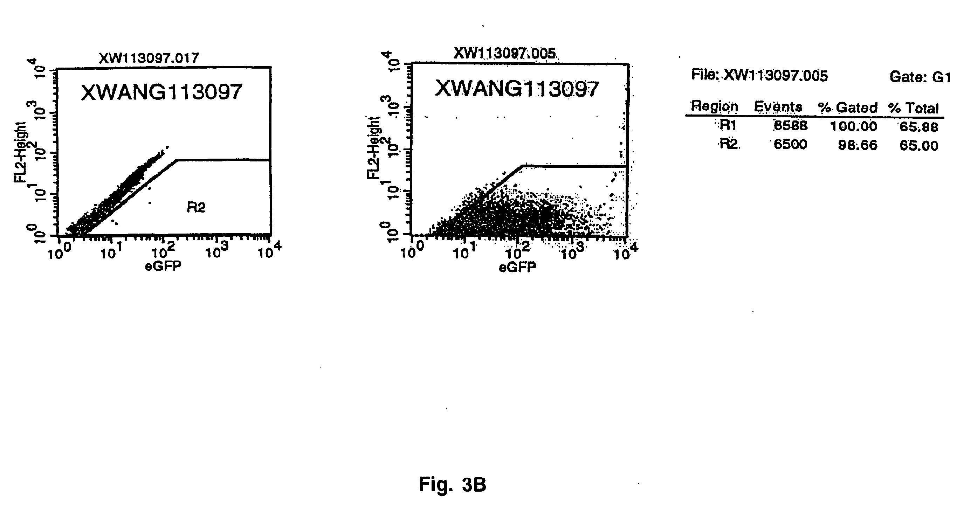

**Figure 3A** demonstrates lentiviral transduction of cultured retinal pigment epithelial cells. Marker gene (eGFP) expression results in green, fluorescent cells. **Figure 3B** shows fluorescent-activated cell sorting analysis of transduction efficiency. Data outside of R2 gate in first panel reflects pre-transduction lack of fluorescence. Second panel demonstrates a post-transduction shift to >95% fluorescence.

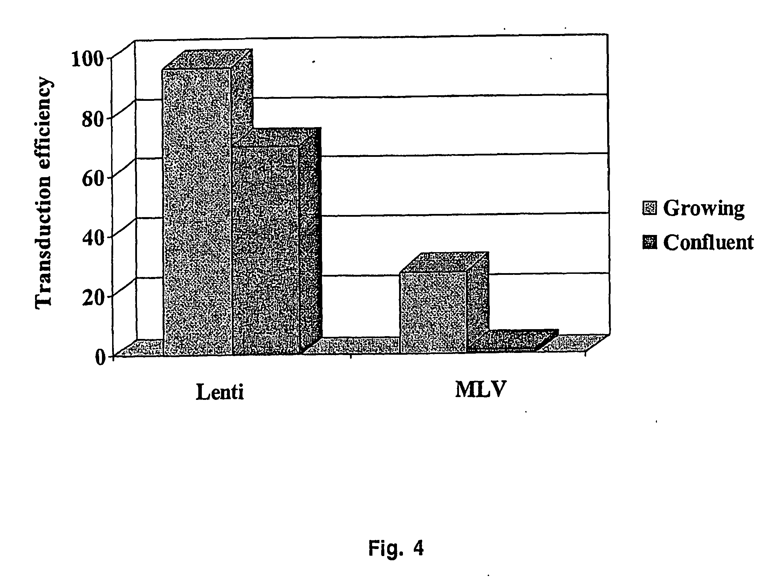

**Figure 4** illustrates mitotic activity and transduction efficiency in human retinal pigment epithelial cells. Human retinal pigment epithelial cells were transduced by lentiviral or murine leukemia viral (MLV) vectors. Cells were mitotically inactive (confluent) or mitotically active (growing) at the time of exposure to vector. These results shown in **Figure 4** demonstrate the superior ability of lentiviral vectors over other retroviral vectors to transduce non-dividing cells.

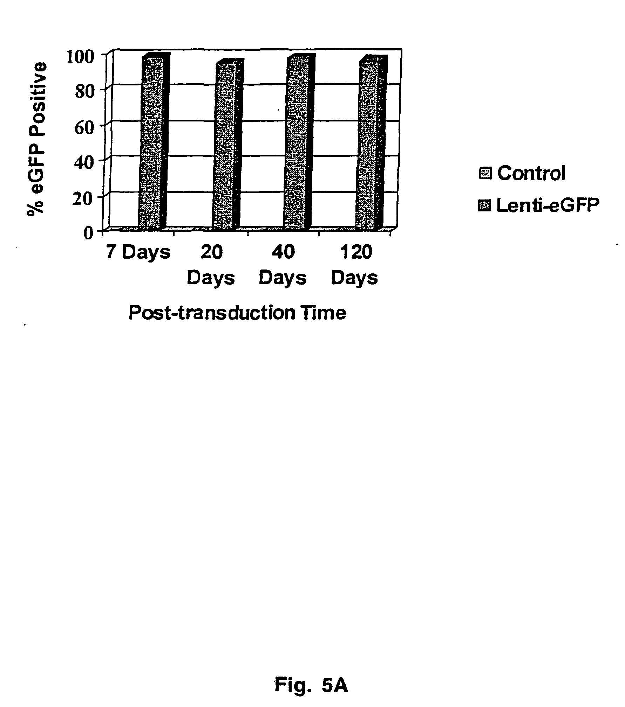

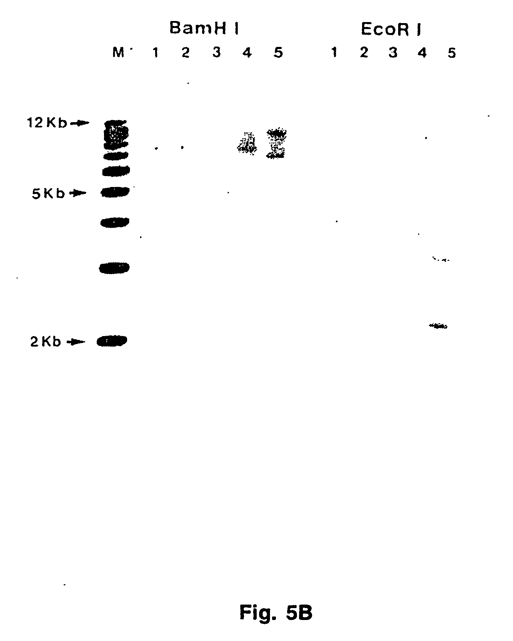

**Figure 5** depicts expression stability in human retinal pigment epithelial cells. Cells were exposed to eGFP-containing lentiviral vectors and were subsequently maintained for at least 120 days in continuous culture. **Figure 5A** depicts the stability of eGFP expression in these cells as well as a lack of selection for, or against, lentivirally transduced cells (the fraction of transduced cells remains constant over time). **Figure 5B** is the result of Southern analysis on 5 clonal populations of cells. Lane 1 contains genomic DNA from the non-transduce parental line. Lanes 2 and 3 contain DNA from cells which were exposed to vector but were not green (non-transduces). Lanes 4 and 5 contain DNA from transduced, green cells. Cells remain e-GFP positive as the result of genomic integration.

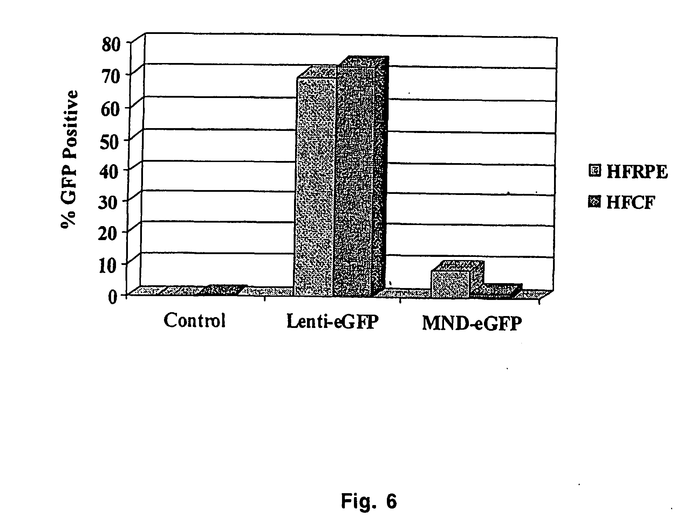

**Figure 6** illustrates human fetal cell transgene expression. This graph depicts the highly efficient mode of transduction achieved with lentiviral vectors when compared with a non-lentiviral retroviral vector (MND-eGFP) or no viral vector (control) in human fetal cells.

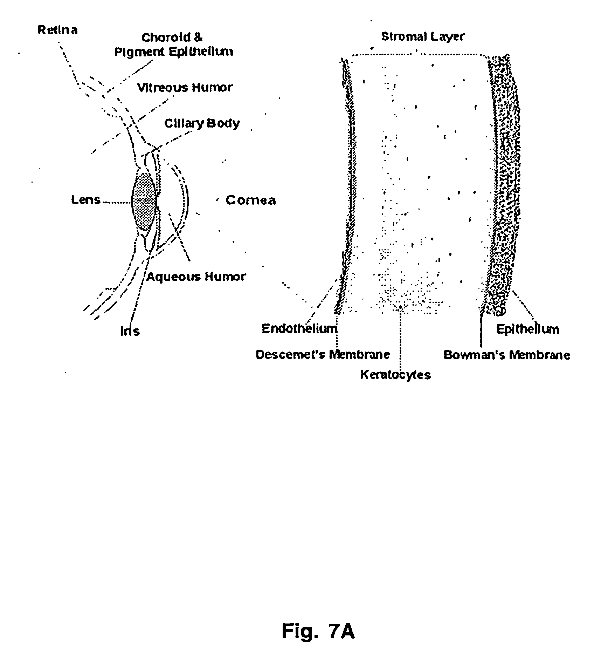

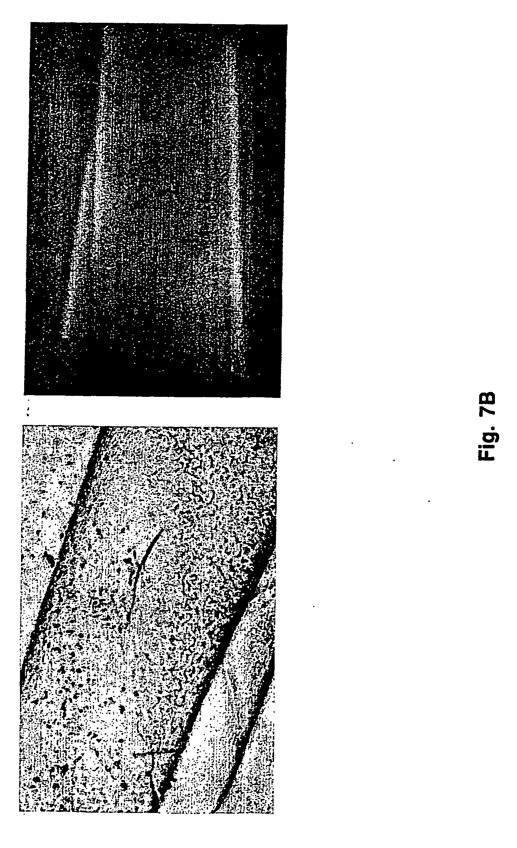

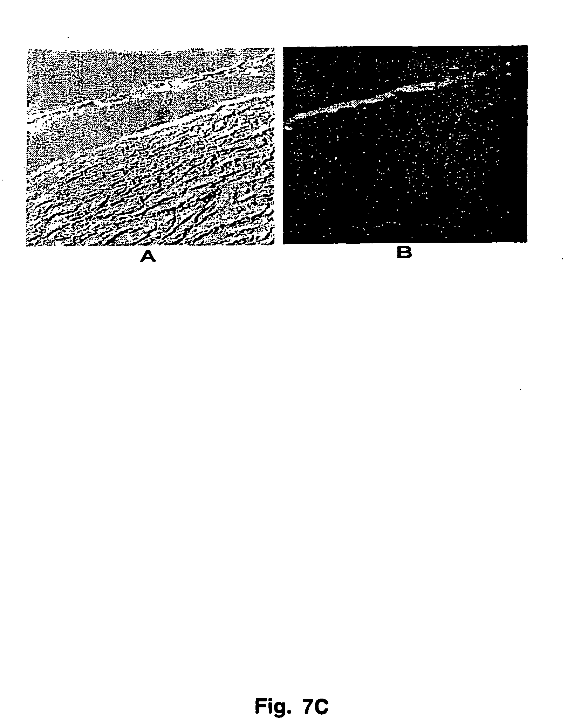

**Figure 7** demonstrates corneal transduction. **Figure 7A** is a schematic representation of the human cornea. **Figure 7B** demonstrates human corneal endothelial transduction by an e-GFP-containing lentiviral vectors. Human corneal buttons, removed at the time of corneal transplant, were exposed to lentiviral particles. Descemet's membrane was subsequently removed and photographed in room light (left) and under conditions amenable to fluorescence detection (right). **Figure 7C** demonstrates lentiviral-mediated eGFP gene transfer to human corneal epithelial cells. Subpanel A is a light micrograph of a human cornea with an artifactually detached epithelial layer. Fluorescent microscopy (subpanel B) reveals epithelial fluorescence.

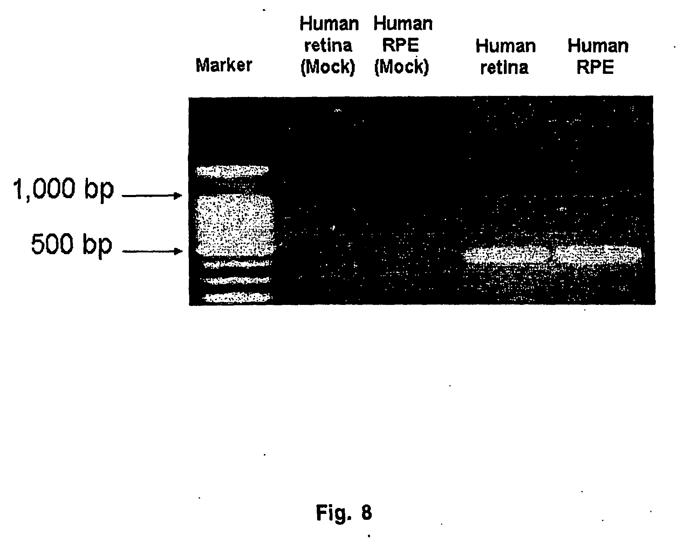

**Figure 8** provides an example of lentiviral gene transfer of a gene whose deficiency results in human disease. Normal human retinal or retinal pigment epithelial (RPE) tissue, surgically excised at the time of enucleation for retinoblastoma, was exposed to lentiviral vectors which either lacked a therapeutic gene (Mock) or contained the human peripherin gene. This gene, when genetically deficient in humans is known to result in a wide variety of disabling phenotypes. Results of reverse transcriptase-assisted polymerase chain reaction (rt-PCR) employing primers designed to recognize only the transferred peripherin gene were shown. The expression of human peripherin in human retinal and retinal pigment epithelial was clearly demonstrated.

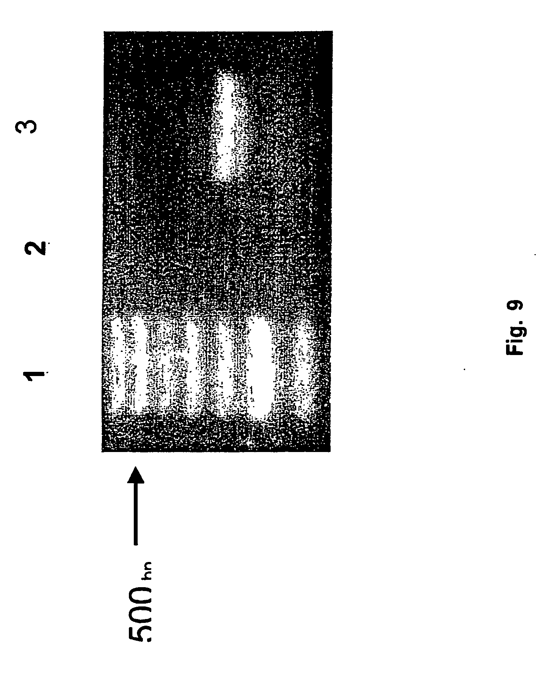

**Figure 9** demonstrates lentiviral-mediated expression of CA-rb mRNA. This shows the results of a reverse transcriptase-assisted polymerase chain reaction (rt-PCR) employing primers designed to recognize only the constitutively active form of the retinoblastoma gene. Lane 1 : marker, Lane 2: reaction results with RNA isolated from lentiviral-eGFP transduced cells, Lane 3: reaction results with RNA isolated from lentiviral-CA-rb transduced cells. The reaction product was of the expected size.

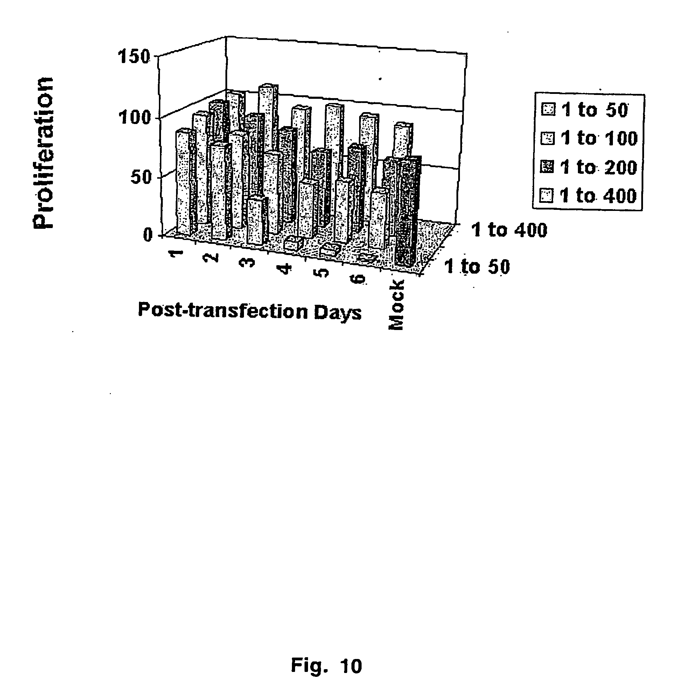

**Figure 10** shows the inhibitory effect of lentiviral constitutively active retinoblastoma gene vector on human retinal and choroidal cell division. Cells were exposed to decreasing dilutions of a single lentiviral stock (1:400 dilution to 1:50 dilution) and growth was compared with cells exposed to lentiviral vectors which did not contain the constitutively active retinoblastoma gene. An inhibitory effect on cell division was clearly seen over time and this effect was dose-dependant.

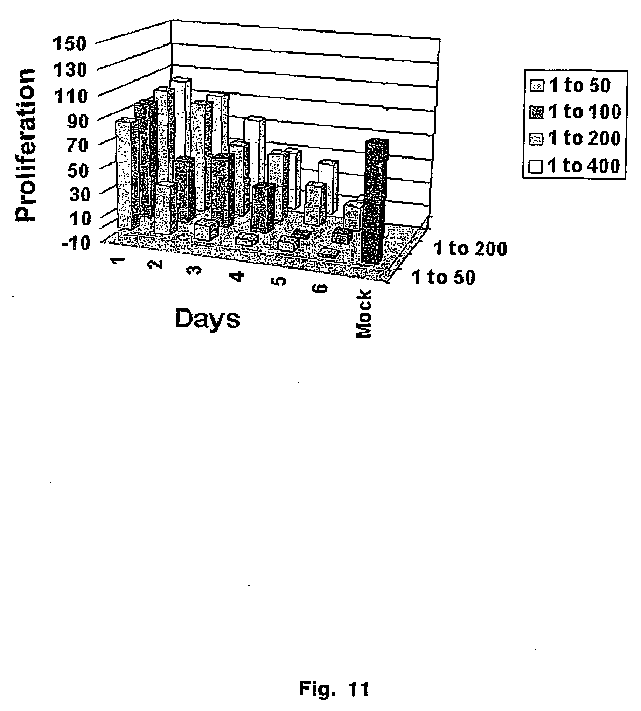

**Figure 11** shows the inhibitory effect of lentiviral CA-rb on human lens epithelial cell division. Cells removed from human eyes at the time of cataract extraction were exposed to decreasing dilutions of a single lentiviral stock (1:400 dilution to 1:50 dilution) and growth was compared with cells exposed to lentiviral vectors which did not contain the constitutively active retinoblastoma gene. An inhibitory effect on cell division was clearly seen over time and this effect was dose-dependant.

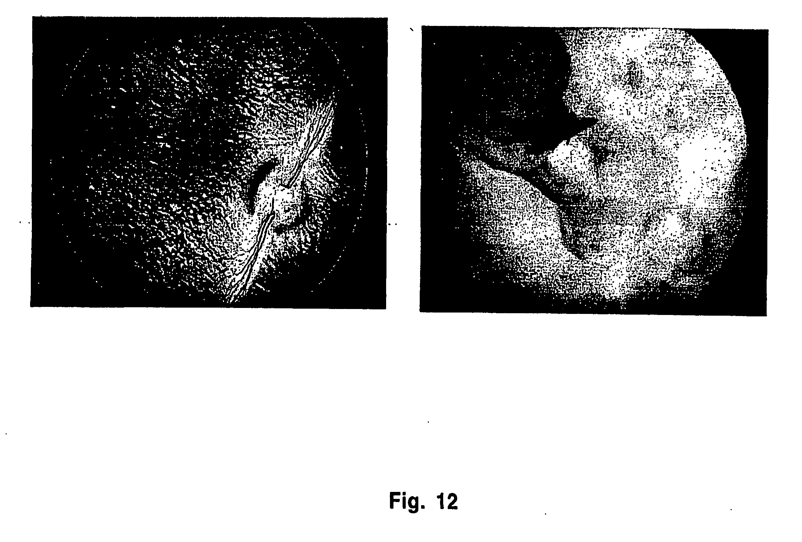

**Figure 12** shows the *in vivo* inhibitory effects of lentiviral CA-rb on blinding intraocular cellular proliferation. Proliferative vitreoretinopathy was induced in three sets of rabbits. One set was not treated, one set was treated with lentiviral vectors lacking the constitutively active retinoblastoma gene and the last set was treated with intravitreally-delivered lentiviral CA-rb. Proliferative vitreoretinopathy and retinal detachment was noted in the first two sets at high frequency (>90%). The fraction of animals that went on to retinal detachment was significantly lower in the set treated with constitutively active retinoblastoma gene (26%). Shown here are two retinal photographs. The eye on the left had a completely attached retina and was treated with a constitutively active retinoblastoma gene. The eye on the right had a completely detached retina, the consequence of intraocular vitreoretinopathic cellular proliferation, and was treated with lentiviral vectors lacking the CA-rb gene.

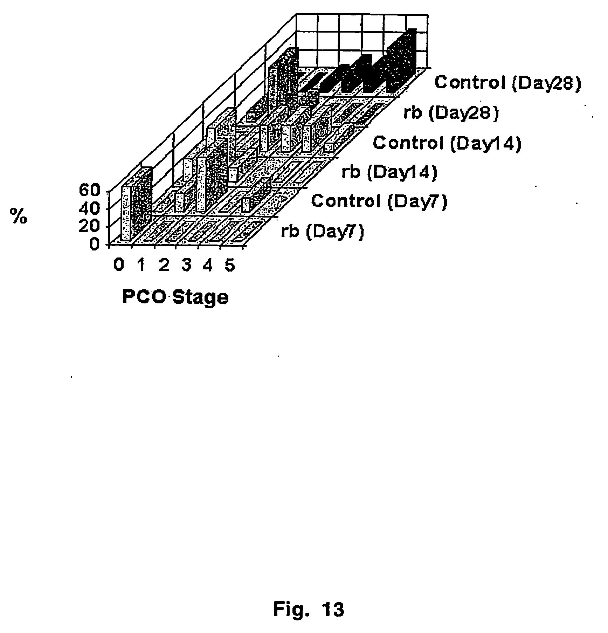

**Figure 13** shows the *in vivo* inhibitory effect of lentiviral CA-rb on the process of post-lens extraction posterior capsular opacification. Three sets of rabbits underwent standard phacoemulsfication to remove the native crystalline lens. The first set (group 1) was subsequently treated with nothing and the second two sets were treated with either empty lentiviral constructs (no therapeutic gene, group 2) or with lentiviral CA-rb (group3) delivered into the intact lens capsular bag at the time of closure of the cataract wound. Animals were serially examined for the presence of posterior capsular opacification. The presence of opacification was graded on a 1 to 5 scale where 1 represented no opacification and 5 represented opacification severe enough to preclude visualization of the retina with indirect binocular ophthalmoscopy. There were no statistically different results obtained between groups 1 and 2 (no treatment and empty vector). The graph here shows a striking inhibitory effect of lentiviral CA-rb on the development of posterior capsule opacification. By day 28, control animals had an average opacification score of 4.4 while animals treated with lentiviral CA-rb had an average opacification score of 2.1.

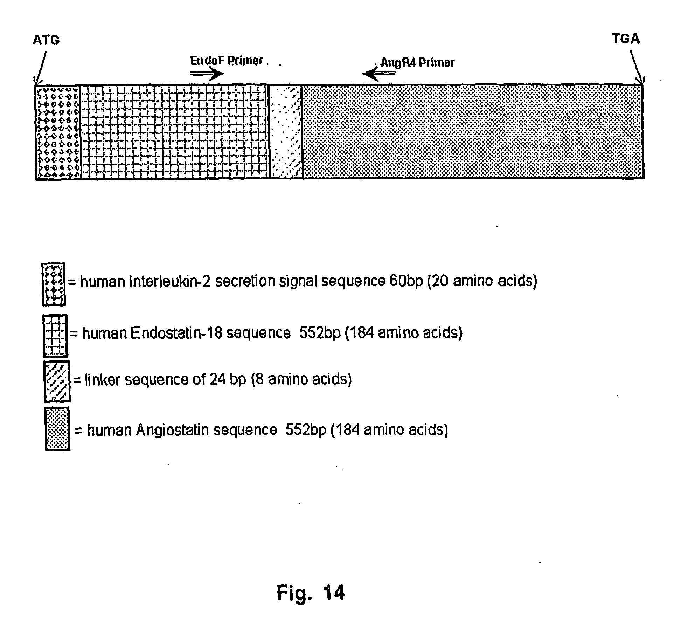

**Figure 14** shows a map for a endostatin-18/angiostatin fusion gene delivered by lentiviral vector.
**Figure 1** **5** shows a map for the lentiviral vector pHR-CMV-Endo/Ang-ires-eGFP carrying an endostatin/angiostatin fusion gene.
**Figure 16** shows a map for the lentiviral vector pHR-CMV-BIK-ires-eGFP carrying a BIK gene.
**Figure 17** shows a map for the lentiviral vector pHR-CMV-Endo/Kringle-ires-eGFP carrying an endostatin/kringle fusion gene.
**Figure 18** shows a map for the lentiviral vector pHR-CMV-KDR-ires-eGFP carrying a KDR gene.
**Figure 19** shows a map for the lentiviral vector pHR-CMV-P16-ires-eGFP carrying a p16 gene.
**Figure 20** shows a map for the lentiviral vector pHR-CMV-P21-ires-eGFP carrying a p21 gene.
**Figure 2** **1** shows a map for the lentiviral vector pHR-CMV-Timp1-ires-eGFP carrying a Timp1 gene.
**Figure 2 2** shows a map for the lentiviral vector pHR-EF1/HTLV-Ang-ires-eGFP carrying an angiostatin gene.
**Figure 2** **3** shows a map for the lentiviral vector pHR-EF1/HTLV-Endo XV-ires-eGFP carrying an endostatin XV gene.
**Figure 2** **4** shows a map for the lentiviral vector pHR-EF1/HTLV-EndoAng-ires-eGFP carrying an endostatin/angiostatin fusion gene.
**Figure 2** **5** shows a map for the lentiviral vector pHR-EF1/HTLV-EndoKringle-ires-eGFP carrying an endostatin/kringle fusion gene.
**Figure 2** **6** shows a map for the lentiviral vector pHR-EF1/HTLV-Kringle 1-5-ires-eGFP carrying a Kringle gene.
**Figure 2** **7** shows a map for the lentiviral vector pHR-EF1/HTLV-MigIP10-ires-eGFP carrying a Mig/IP10 fusion gene.
**Figure 2** **8** shows a map for the lentiviral vector pHR-EF1/HTLV-Timp1-ires-eGFP carrying a Timp1 gene.
**Figure 2** **9** shows a map for the lentiviral vector pHR-EF1/HTLV-Timp4-ires-eGFP carrying a Timp4 gene.

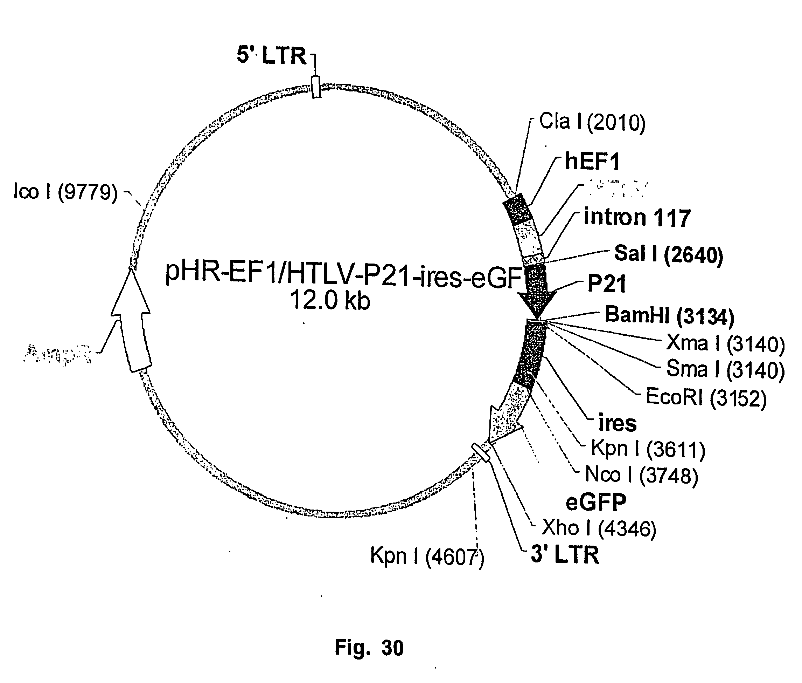

**Figure 30** shows a map for the lentiviral vector pHR-EF1/HTLV-P21-ires-eGFP carrying a p21 gene.
**Figure 3** **1** shows a map for the lentiviral vector pHR-EF1/HTLV-Endo XVIII-ires-eGFP carrying an endostatin XVIII gene.

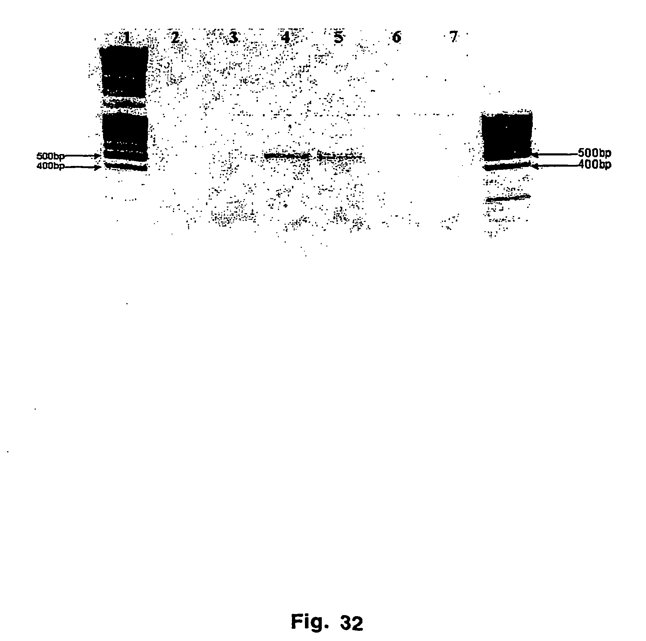

**Figure 32** shows RT-PCR of mRNA isolated from human dermal microvascular endothelial (hDMVE) cells transduced with the endostatin-18/angiostatin fusion gene. Lanel: 1000/100 bp ladder mix; lane 2-5: RT-PCR from mRNA isolated from hDMVE cells transduced with 1 ul, 5 ul, 10 ul and 20 ul of pHR'-eF1α/HTLV-Endo::Ang-IRES-eGFP virus supernatant from a single well of a 12 well plate; lane 6: RT-PCR from mRNA isolated from hDMVE cells incubated with 20 µl of PBS; lane 7: negative control (H2O as template for RT-PCR); lane 8: 100 bp ladder.

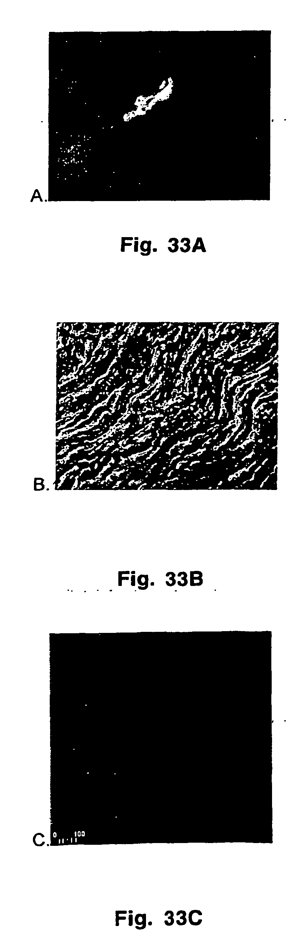

**Figure 3 3** shows the presence of eGFP in the corneal micropocket in treated animals. **Figure 33A** shows a fluorescent photomicrograph demonstrating the presence of eGFP expression in a micropocket. **Figure 33B** shows a non-fluorescent photomicrograph of the same tissue as shown in **Figure 33A. Figure 33C** shows a fluorescent photomicrograph of a similarly processed tissue from an untreated animal.

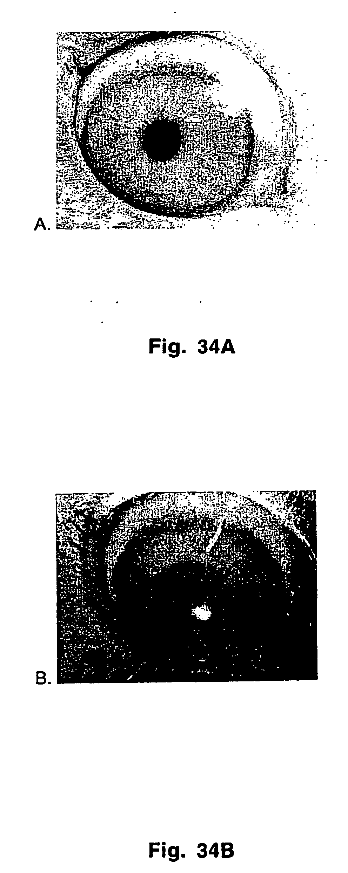

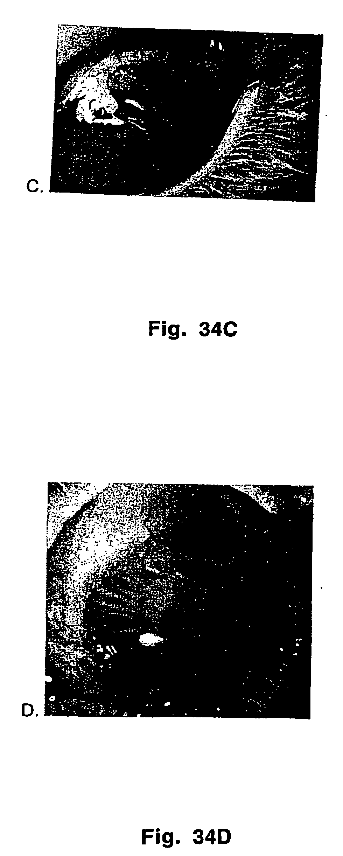

**Figure 34** shows an inhibitory effect on neovascularization in animals treated with a Mig/IP10 lentiviral vector. **Figure 34A** shows a photograph of normal (nontreated, nonstimulated) cornea. **Figure 34B** shows a photograph of an alkali challenged cornea of an animal treated with a Mig/IP10 lentiviral vector. Note the lack of blood vessels into the cornea. **Figure 34C** shows a photograph of an alkali challenged cornea of an animal treated with a control lentiviral vector without a therapeutic anti-angiogenic gene. Note the invasion of blood vessels into the cornea. **Figure 34D** shows a photograph of an alkali challenged cornea of an untreated animal. Note the invasion of blood vessels into the cornea.

### DETAILED DESCRIPTION OF THE INVENTION

Lentiviruses are slow viruses whose natural pathogenicity occurs over a period of months to years. This viral genus includes such retroviruses as HIV. These viruses are known to infect and transduce a wide variety of terminally differentiated, mitotically active or inactive human cell types. Their transduction efficiency is very high, even cell lines traditionally very refractory to gene transfer such as human retinal, corneal, trabecular, lenticular, retinal pigment epithelial, proliferative vitreoretinopathic and vascular endothelial cells can be transduced using this vector.

Upon infection with the lentivirus, the viral genetic material integrates itself within the host genome. Thus, the viral genes become a permanent part of the host cell's genetic material and gene expression is constant for the life of the cell. Each cell transduced by a lentivirus will transmit the genetic information to its progeny. The use of lentiviruses as vectors in gene therapy for intraocular diseases is possible since under natural conditions of infection with the parental virus, the virus is an intraocular pathogen that is not associated with an inflammatory response. Previous work with this virus has demonstrated its successful use in transduction of both neural and retinal cells (Naldini et al., 1996; Miyoshi et al., 1997).

The present invention provides a new lentiviral vector that incorporated an IRES (internal ribosome entry site) element between two cloning sites. The IRES element allows mRNA-ribosome binding and protein synthesis. This backbone can accommodate two different expressible genes. A single message is produced in transduced cells; however, because of the IRES element, this message is functionally bi-cistronic and can drive the synthesis of two different proteins. These two genes are placed under the control of strong promoters such as CMV or HTLV promoters. Alternatively, one of skill in the art would readily employ other promoters known to be active in human retinal, corneal or retinal pigment epithelial cells. In this fashion each of the potentially therapeutic genes discussed below can be linked to a marker gene (e.g. the enhanced green fluorescent gene - eGFP gene) so that transduced cells will simultaneously be marked and able to express the therapeutic gene of interest. Marked cells can easily be isolated *in vitro* and observed *in vivo.* It would be apparent to one of skill in the art that other marker genes besides the enhanced green fluorescent protein gene could be incorporated into the lentiviral vector. Since the level of ordinary skill of an average scientist in the areas of genetic engineering and cloning has increased substantially in recent years, a person having ordinary skill in this art would readily be able to construct lentiviral vectors containing other therapeutic genes of interest in addition to those disclosed herein. Moreover, the lentiviral vector system disclosed herein can transfer genes known to be deficient in human patients with inherited eye disease or other diseases. The transfer of these genes to human ocular cells or other tissues by this system forms the basis for useful therapies for patients with various diseases.

The basic discovery detailed herein demonstrates that lentiviral vectors can transfer a variety of genes to modify abnormal intraocular proliferation and, hence, decrease the incidence of neovascular disease, retinal detachment or post-cataract extraction posterior capsular opacification. A number of therapeutic genes may be useful in clinical circumstances for in vivo inhibition of intraocular cell division. These genes include a variety of recently identified modulators for the process of new blood vessel growth (angiogenesis) or apoptosis. It is believe that genetic control of the expression of these modulators via lentivirus-mediated gene transfer would prove useful in the treatment of intraocular neovascular diseases such as age-related macular degeneration (AMD), retinopathy of prematurity (ROP) and proliferative diabetic retinopathy (PDR).

The lentiviral vectors disclosed herein can readily be applied in clinical settings.

Vascular endothelial cells play a central role in both vasculogenesis and angiogenesis. These cells respond mitogenically (become active with regards to cell division or migration) to a variety of protein cytokines. For example, vascular endothelial growth factor (VEGF), angiogenin, angiopoietin-1 (Ang1) and angiotropin are cytokines that stimulate endothelial cell division, migration or cell-cell adhesion, and thus favor the process of angiogenesis. Endostatin, soluble (decoy) VEGF receptors (sflt), and thrombospondin are endogenous protein cytokines that appear to inhibit angiogenesis. The present invention demonstrates that many of these inhibitory proteins delivered by lentiviral vectors are useful in the treatment of intraocular neovascularization. Examples of genes that can be incorporated into the lentiviral vectors of the present invention include, but are not limited to, the following genes:

### TISSUE INHIBITORS OF METALLOPROTEINASES

The tissue inhibitors of metalloproteinases (TIMPs) represent a family of ubiquitous proteins that are natural inhibitors of the matrix metalloproteinases (MMPs). Matrix metalloproteinases are a group of zinc-binding endopeptidases involved in connective tissue matrix remodeling and degradation of the extracellular matrix (ECM), an essential step in tumor invasion, angiogenesis, and metastasis. The MMPs each have different substrate specificities within the ECM and are important in its degradation. The analysis of MMPs in human mammary pathology showed that several MMPs were involved in degradation of the ECM: collagenase (MMP1) degrades fibrillar interstitial collagens; gelatinase (MMP2) mainly degrades type IV collagen; and stromelysin (MMP3) has a wider range of action (Bramhall et al., 1996, 1997). There are four members of the TIMP family. TIMP-1 and TIMP-2 are capable of inhibiting tumor growth, invasion, and metastasis that has been related to MMP inhibitory activity. Furthermore, both TIMP-1 and TIMP-2 are involved with the inhibition of angiogenesis. Unlike other members of the TIMP family, TIMP-3 is found only in the ECM and may function as a marker for terminal differentiation. Finally, TIMP-4 is thought to function in a tissue-specific fashion in extracellular matrix hemostasis (Gomez et al., 1997).

### TIMP-1

Tissue inhibitor of metalloproteinase-1 (TIMP-1) is a 23kD protein that is also known as metalloproteinase inhibitor 1, fibroblast collagenase inhibitor, collagenase inhibitor and erythroid potentiating activity (EPA). The gene encoding TIMP-1 has been described by Docherty et al. (1985). TIMP-1 complexes with metalloproteinases (such as collagenases) causing an irreversible inactivation. The effects of TIMP-1 have been investigated in transgenic mouse models: one that overexpressed TIMP-1 in the liver, and another that expressed the viral oncogene Simian Virus 40/T antigen (TAg) leading to heritable development of hepatocellular carcinomas. In double transgenic experiments (the TIMP-1 lines were crossed with the TAg transgenic line), overexpression of hepatic TIMP-1 was reported to block the development of TAg-induced hepatocellular carcinomas by inhibiting growth and angiogenesis (Martin et al., 1996).

### TIMP-2

Tissue inhibitor of metalloproteinase-2 (TIMP-2) is a 24kD protein that is also known as metalloproteinase inhibitor 2. The gene encoding TIMP-2 has been described by Stetler-Stevenson et al. (1990). Metalloproteinase (MMP2) which plays a critical role in tumor invasion is complexed and inhibited by TIMP-2. Thus, TIMP-2 could be useful to inhibit cancer metastasis (Musso et al., 1997). When B16F10 murine melanoma cells (a highly invasive and metastatic cell line) were transfected with a plasmid coding for human TIMP-2 and injected subcutaneously in mice, TIMP-2 over-expression limited tumor growth and neoangiogenesis in vivo (Valente et al., 1998).

### TIMP-3

Tissue inhibitor of metalloproteinase-3 (TIMP-3) is also known as metalloproteinase inhibitor 3. When breast carcinoma and malignant melanoma cell lines were transfected with TIMP-3 plasmids and injected subcutaneously into nude mice, suppression of tumor growth was observed (Anand-Apte et al., 1996). However, TIMP-3 over-expression had no effect on the growth of the two tumor cell lines in vitro. Thus, it was suggested that the TIMP-3 released to the adjacent ECM by tumor cells inhibited tumor growth by suppressing the release of growth factors sequestered in ECM, or by inhibiting angiogenesis (Anand-Apte et al., 1996).

### TIMP-4

Tissue inhibitor of metalloproteinase-4 (TIMP-4) is also known as metalloproteinase inhibitor 4. The TIMP-4 gene and tissue localization have been described by Greene et al. (1996). Biochemical studies have shown that TIMP-4 binds human gelatinase A similar to that of TIMP-2 (Bigg et al., 1997). The effect of TIMP-4 modulation on the growth of human breast cancers in vivo was investigated by Wang et al. (1997). Overexpression of TIMP-4 was found to inhibit cell invasiveness in vitro, and tumor growth was significantly reduced following injection of nude mice with TIMP-4 tumor cell transfectants in vivo (Wang et al., 1997).

### ENDOSTATIN, ANGIOSTATIN, PEX, KRINGLE-5 AND FUSION GENES

J. Folkman and his colleagues (Boehm et al., 1997) showed that treatment of mice with Lewis lung carcinomas with the combination of endostatin + angiostatin proteins induced the complete regression of the tumors, and that mice remained healthy for the rest of their life. This effect was obtained only after one cycle (25 days) of endostatin + angiostatin treatment, whereas endostatin alone required 6 cycles to induce tumor dormancy.

D. Hanahan and colleagues (Bergers et al., 1999) demonstrated a superior antitumoral effect of the combination of endostatin + angiostatin proteins in a mouse model for pancreatic islet carcinoma. Endostatin + angiostatin combination resulted in a significant regression of the tumors, whereas endostatin or angiostatin alone had no effect.

### ENDOSTATIN XVIII

Endostatin, an angiogenesis inhibitor produced by hemangioendothelioma, was first identified by O'Reilly et al. (1997). Endostatin is a 20kD C-terminal fragment of collagen XVIII that specifically inhibits endothelial proliferation, and potently inhibits angiogenesis and tumor growth. In fact, primary tumors have been shown to regress to dormant microscopic lesions following the administration of recombinant endostatin (O'Reilly et al., 1997). Endostatin is reported to inhibit angiogenesis by binding to the heparin sulfate proteoglycans involved in growth factor signaling (Zetter, 1998).

### ENDOSTATIN XV

Recently, a C-terminal fragment of collagen XV (Endostatin XV) has been shown to inhibit angiogenesis like Endostatin XVIII, but with several functional differences (Sasaki et al., 2000).

### ANGIOSTATIN

Angiostatin, an internal fragment of plasminogen comprising the first four kringle structures, is one of the most potent endogenous angiogenesis inhibitors described to date. It has been shown that systemic administration of angiostatin efficiently suppresses malignant glioma growth in vivo (Kirsch et al., 1998). Angiostatin has also been combined with conventional radiotherapy resulting in increased tumor eradication without increasing toxic effects in vivo (Mauceri et al., 1998). Other studies have demonstrated that retroviral and adenoviral mediated gene transfer of angiostatin cDNA resulted in the inhibition of endothelial cell growth in vitro and angiogenesis in vivo. The inhibition of tumor-induced angiogenesis produced an increase in tumor cell death (Tanaka et al., 1998). Gene transfer of a cDNA coding for mouse angiostatin into murine T241 fibrosarcoma cells has been shown to suppress primary and metastatic tumor growth in vivo (Cao et al., 1998).

### PEX

PEX is the C-terminal hemopexin domain of MMP-2 that inhibits the binding of MMP-2 to integrin alphavbeta3 blocking cell surface collagenolytic activity required for angiogenesis and tumor growth was cloned and described by Brooks et al. (1998).

### KRINGLE-5

The kringle-5 domain of human plasminogen, which shares high sequence homology with the four kringles of angiostatin, has been shown to be a specific inhibitor for endothelial cell proliferation. Kringle-5 appears to be more potent than angiostatin on inhibition of basic fibroblast growth factor-stimulated capillary endothelial cell proliferation (Cao et al., 1997). In addition to its antiproliferative properties, kringle-5 also displays an antimigratory activity similar to that of angiostatin, which selectively affects endothelial cells (Ji et al., 1998).

### ANGIOSTATIC FUSION GENES

Novel angiostatic fusion genes can be cloned using an elastin peptide motif (Val-Pro-Gly-Val-Gly) as a linker. These fusions combine two potent angiostatic genes to increase the suppression of tumor angiogenesis. Since these molecules operate through different mechanisms, their combination may result in synergistic effects. Examples of angiostatic fusion proteins include, but are not limited to, the fusion of endostatin 18 and angiostatin (endo/ang), endostatin18 and the kringle 5 motif of plasminogen (endo/k5) as well as the monokine-induced by interferon-gamma and the interferon-alpha inducible protein 10 (MIG/IP10).

### CHEMOKINES

Chemokines are low-molecular weight pro-inflammatory cytokines capable of eliciting leukocyte chemotaxis. Depending on the chemokine considered, the chemoattraction is specific for certain leukocytes cell types. Expressing chemokine genes into tumors may lead to more efficient recruiting of leukocytes capable of antitumoral activity. Moreover, in addition to their chemotactic activity, some chemokines possess an anti-angiogenic activity: they inhibit the formation of blood vessels feeding the tumor. For this reason, these chemokines are useful in cancer treatment.

### MIG

Mig, the monokine-induced by interferon-gamma, is a CXC chemokine related to IP-10 and produced by monocytes. Mig is a chemoattractant for activated T cells, and also possesses strong angiostatic properties. Intratumoral injections of Mig induced tumor necrosis (Sgadari et al., 1997).

### IP-10

IP-10, the interferon-alpha inducible protein 10, is a member of the CXC chemokine family. IP-10 is produced mainly by monocytes, but also by T cells, fibroblasts and endothelial cells. IP-10 exerts a chemotactic activity on lymphoid cells such as T cells, monocytes and NK cells. IP-10 is also a potent inhibitor of angiogenesis: it inhibits neovascularization by suppressing endothelial cell differentiation. Because of its chemotactic activity toward immune cells, IP-10 was considered as a good candidate to enhance antitumour immune responses. Gene transfer of IP-10 into tumor cells reduced their tumorigenicity and elicited a long-term protective immune response (Luster and Leder, 1993). The angiostatic activity of IP-10 was also shown to mediate tumor regression: tumor cells expressing IP-10 became necrotic in vivo (Sgadari et al., 1996). IP-10 was also shown to mediate the angiostatic effects of IL-12 that lead to tumor regression (Tannenbaum et al., 1998).

### SOLUBLE VEGF RECEPTORS

FLT-1 (fms-like tyrosine kinase 1 receptor) is a membrane-bound receptor of VEGF (VEGF Receptor 1). It has been shown that a soluble fragment of FLT-1 (sFLT-1) has angiostatic properties by way of its antagonist activity against VEGF. Soluble FLT-1 acts by binding to VEGF but also because it binds and blocks the external domain of the membrane-bound FLT-1 (Kendall & Thomas 1993, Goldman et al., 1998). One example of sFLT-1 is a human sFLT-1 spanning the 7 immunoglobulin-like domains of the external part of FLT-1.

### sFLK-1 / KDR

FLK-1 or KDR (kinase insert domain receptor) is a membrane-bound receptor of VEGF (VEGF Receptor 2). It has been shown that a soluble fragment of KDR (sKDR) has angiostatic properties by way of its antagonist activity against VEGF, probably because it binds VEGF but also because it binds and blocks the external domain of the membrane-bound KDR (Kendall & Thomas. 1996, Millauer et al. 1994). One example of sKDR is a human sKDR spanning the 7 immunoglobulin-like domains of the external part of KDR.

### APOPTOSIS

Apoptosis is the term used to describe the process of programmed cell death or cell suicide. This process is a normal component of the development and health of multicellular organisms. The abnormal regulation of apoptosis has been implicated in a variety of pathological disorders from cancer to autoimmune diseases.

### BIK

Bik is a 18kD (160 amino acids) potent pro-apoptotic protein, also known as Bcl-2 interacting killer, apoptosis inducer NBK, BP4, and BIP1. Bik is encoded by the gene bik (or nbk). The function of Bik is to accelerate programmed cell death by complexing with various apoptosis repressors such as Bc1-XL, BHRF1, Bcl-2, or its adenovirus homologue E1B protein. In transient transfection studies, Bik promoted cell death in a manner similar to the pro-apoptotic members of the Bcl-2 family, Bax and Bak (Boyd et al., 1995).

### BAK

Bak, a Bcl-2 homologue, is a pro-apoptotic protein that promotes apoptosis by binding anti-apoptotic family members including Bcl-2 and Bc1-XL and inhibits their activity as previously described for Bik (Chittenden et al., 1995).

### BAX

Bax is a 21kD protein that functions as an apoptosis regulator. Bax accelerates programmed cell death by dimerizing with and antagonizing the apoptosis repressor Bcl-2. The ratio of these protein dimers is thought to relate to the initiation of apoptosis. The effect of recombinant Bax expression in K562 erythroleukemia cells has been investigated by Kobayashi et al. (1998). Transfection with the Bax vector into K562 cells resulted in the induction of apoptosis. Furthermore, cells stably transfected with Bax were found to be more sensitive to the chemotherapeutic agents ara-X, doxorubicin, and SN-38 (Kobayashi et al., 1998).

### BAD

The Bad protein (Bcl-2 binding component 6, bad gene or bbc6 or bc1218) is a small protein (168 amino acids, 18kDa) which promotes cell death. It successfully competes for the binding to Bc1-XL and Bcl-2, thereby affecting the level of heterodimerization of both these proteins with Bax. It can reverse the death repressor activity of Bc1-XL, but not that of Bcl-2.

### BCL-2

B cell leukemia/lymphoma-2 (Bcl-2) is the prototype member of a family of cell death regulatory proteins. Bcl-2 is found mainly in the mitochondria and blocks apoptosis by interfering with the activation of caspases. Gene transfer of Bcl-2 into tumor cells has been shown to enhance their metastatic potential (Miyake et al., 1999). Bcl-2 gene transfer may be applied to bone marrow transplant since Bcl-2 enhances the survival of hematopoietic stem cells after reconstitution of irradiated recipient (Innes et al., 1999). Also, Bcl-2 gene transfer could be useful against neurodegenerating diseases since expression of Bcl-2 in neurons protects them from apoptosis (Saille et al., 1999).

### BCL-XS

Bcl-XS (short isoform) is a dominant negative repressor of Bcl-2 and Bcl-XL. It has been used in gene therapy experiments to initiate apoptos is in tumors that express Bcl-2 and Bcl-XL. Expression of Bcl-XS reduces tumor size (Ealovega et al., 1996) and sensitizes tumor cells to chemotherapeutic agents (Sumatran et al., 1995), suggesting a role for Bcl-XS in initiating cell death in tumors that express Bcl-2 or Bcl-XL (Dole et al., 1996).

### GAX

Gax is an homeobox gene coding for a transcription factor that inhibits cell proliferation in a p21-dependent manner. Gax is down-regulated when cells are stimulated to proliferate. Gax over-expression leads to Bcl-2 down-regulation and Bax up-regulation in mitogen-activated cells (Perlman et al., 1998). Thus, Gax may be useful to inhibit the growth of certain tumor cells. Moreover, Gax over-expression in vascular smooth muscle cells inhibits their proliferation (Perlman et al., 1999). Hence, Gax gene transfer could limit vascular stenosis following vascular injuries.

### TUMOR SUPPRESSOR GENES

Various mutations of tumor suppressor genes have been associated with different types of cancers. In these cases, somatic gene therapy with wild-type versions of tumor suppressor genes have been contemplated as anti-cancer therapeutic approaches. p16, p21, p27 & p53 inhibit the cell cycle by acting on the cyclin-dependent kinases.

### P16

P16, a 15kD protein (148 amino acids), is also known as CDK4I, P16-INK4, P16-INK4A, or multiple tumor suppressor 1 (MTS1). P16 is encoded by the gene cdkn2a or cdkn2. P16 forms a heterodimer with cyclin-dependent kinase 4 and 6, thereby preventing their interaction with cyclin D both in vitro and in vivo. Thus, P16 acts as a negative regulator of the proliferation of normal cells. P16 (cdkn2) mutations are involved in tumor formation in a wide range of tissues. cdkn2a is homozygously deleted, mutated, or otherwise inactivated in a large proportion of tumor cell lines and some primary tumors including melanomas and tumors of the biliary tract, pancreas and stomach (Biden et al., 1997; Castellano et al., 1997). Loss of p16IKN4a gene expression is commonly observed in mesothelioma tumors and other cell lines. It has been shown that p16INK4A transduction with an expressing adenovirus in mesothelioma cells results in a decrease of cell growth, and in the death of the transduced cells (Frizelle et al., 1998). Furthermore, adenoviral mediated gene transfer of wild-type p16 into three human glioma cell lines (U251 MG, U-87 MG and D54 MG) that were not expressing an endogenous p16/CDKN2 gene resulted in the arrest of cell growth in the G0 and G1 phases (Fueyo et al., 1996). In addition, adenoviral mediated gene transfer of wild-type p 16-INK4A into lung cancer cell lines that do not express p16-INK4A inhibited tumor proliferation both in vitro and in vivo (Jin et al., 1995). Thus, the restoration of the wild-type P16 protein in tumor cells could have cancer therapeutic utility.

### P21

p21 is an 18kD protein (164 amino acids) also known as Cyclin-Dependent Kinase Inhibitor 1 (CDKN1), melanoma differentiation associated protein 6 (MDA-6), and CDK-interacting protein 1. p21 is encoded by the gene CDKN1 (Harper et al., 1993), also known as CIP1 and WAF1. p21 may be the important intermediate by which p53 mediates its role as an inhibitor of cellular proliferation in response to DNA damage. p21 may bind to and inhibit cyclin-dependent kinase activity, preventing the phosphorylation of critical cyclin-dependent kinase substrates and blocking cell cycle progression and proliferation. p21 is expressed in all adult human tissues. p21 gene transfer into tumor cells could be useful to inhibit tumor growth. Recombinant adenovirus mediated p21 gene transfer in two human non-small cell lung cancer (NSCLC) cell lines resulted in a dose-dependent p21 induction and concomitant cell growth inhibition due to G0/G1 cell cycle arrest. Moreover, injection of an adenovirus carrying p21 into NSCLC pre-established tumors in mice reduced tumor growth and increased survival of the animals (Joshi et al., 1998). These results support the use of p21 for cancer gene therapy.

In accordance with the present invention, there may be employed conventional molecular biology, microbiology, and recombinant DNA techniques within the skill of the art. Such techniques are explained fully in the literature. See, e.g., Maniatis, Fritsch & Sambrook, "Molecular Cloning: A Laboratory Manual (1982); "DNA Cloning: A Practical Approach," Volumes I and II (D.N. Glover ed. 1985); "Oligonucleotide Synthesis" (M.J. Gait ed. 1984); "Nucleic Acid Hybridization" (B.D. Hames & S.J. Higgins eds. (1985)); "Transcription and Translation" (B.D. Hames & S.J. Higgins eds. (1984)); "Animal Cell Culture" [R.I. Freshney, ed. (1986)); "Immobilized Cells And Enzymes" (IRL Press, (1986)); B. Perbal, "A Practical Guide To Molecular Cloning" (1984).

A "vector" is a replicon, such as plasmid, phage or cosmid, to which another DNA segment may be attached so as to bring about the replication of the attached segment.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes. Various promoters may be used to drive vectors.

A cell has been "transduced" by exogenous or heterologous DNA when such DNA has been introduced inside the cell, usually by a viral vector. The transducing DNA may (as in the case of lentiviral vectors) or may not be integrated (covalently linked) into the genome of the cell. In prokaryotes, yeast, and mammalian cells for example, DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or common ancestor by mitosis. A "cell line" is a clone of a primary cell that is capable of stable growth in vitro for many generations.

A "therapeutic gene" refers to a gene that confers a desired phenotype. For example, a constitutively active retinoblastoma (CA-rb) gene is used to prevent intraocular proliferation or a genetic deficit is restored by the transfer of peripherin gene.

As used herein, the term "marker gene" refers to a coding sequence attached to heterologous promoter or enhancer elements and whose product is easily and quantifiably assayed when the construct is introduced into tissues or cells. Markers commonly employed include radioactive elements, enzymes, proteins (such as the enhanced green fluorescence protein) or chemicals which fluoresce when exposed to ultraviolet light, and others.

The present invention is directed to a novel means of treating inherited or proliferative blinding diseases by means of lentiviral gene transfer. Thus, the present invention includes a lentivirus vector which carries a DNA sequence encoding a gene helpful in the treatment of such a disease. Examples of this include, but are not limited to, the peripherin gene, a constitutively active form of the rb gene and various therapeutic genes discussed above.

The present invention is drawn to a method of inhibiting intraocular cellular proliferation in an individual having an ocular disease, comprising the step of administering to said individual a pharmacologically effective dose of a lentiviral vector comprising a therapeutic gene that inhibits intraocular cellular proliferation. Representative examples of ocular diseases which may be treated using this method of the present invention include age-related macular degeneration, proliferative diabetic retinopathy, retinopathy of prematurity, glaucoma, and proliferative vitreoretinopathy. The therapeutic gene can be a constitutively active form of the retinoblastoma gene, a p16 gene or a p21 gene. Preferably, the lentiviral vector is administered in a dosage of from about 10⁶ to 10⁹ transducing units into the capsular, vitreal or sub-retinal space.

The present invention is also drawn to a method of inhibiting intraocular neovascularization in an individual having an ocular disease, comprising the step of administering to said individual a pharmacologically effective dose of a lentiviral vector comprising a therapeutic gene that inhibits intraocular neovascularization. Representative examples of ocular diseases which may be treated using this method of the present invention include age-related macular degeneration, proliferative diabetic retinopathy, retinopathy of prematurity, glaucoma, and proliferative vitreoretinopathy. The therapeutic gene can be a gene that regulates angiogenesis or apoptosis. In general, genes that regulate angiogenesis include genes that encode tissue inhibitor of metalloproteinase (TIMP)-1, TIMP-2, TIMP-3, TIMP-4, endostatin, angiostatin, endostatin XVIII, endostatin XV, the C-terminal hemopexin domain of matrix metalloproteinase-2, the kringle 5 domain of human plasminogen, a fusion protein of endostatin and angiostatin, a fusion protein of endostatin and the kringle 5 domain of human plasminogen, the monokine-induced by interferon-gamma (Mig), the interferon-alpha inducible protein 10 (IP10), a fusion protein of Mig and IP10, soluble FLT-1 (fms-like tyrosine kinase 1 receptor), and kinase insert domain receptor (KDR), whereas genes that regulate apoptosis include genes that encode Bcl-2, Bad, Bak, Bax, Bik, Bcl-X short isoform and Gax. Preferably, the lentiviral vector is administered in a dosage of from about 10⁶ to 10⁹ transducing units into the capsular, vitreal or sub-retinal space.

The following examples are given for the purpose of illustrating various embodiments of the invention and are not meant to limit the present invention in any fashion:

### EXAMPLE 1

### Cells And Tissue

Primary explants of human choroidal fibroblasts (HCF), human umbilical vein endothelial cells (HUVEC) and human fetal retinal pigment epithelial cells (HRPE) were established and were plated in conditions which either did or did not promote mitotic activity. Stable photoreceptor-derived cells (Y-79 and Weri-Rb-1) were also cultured.

Human retina and RPE, obtained at the time of enucleation for retinoblastoma were used to demonstrate the ability of lentiviral vectors to transduce these mitotically inactive cells and induce the expression of an exogenous human peripherin transgene. Human corneas obtained at the time of corneal transplant surgery were used to demonstrate the ability of lentiviral vectors to transduce these mitotically inactive cells with the marker gene enhanced green fluorescence protein gene.

### EXAMPLE 2

### Lentivirus Vector

A three plasmid-based lentiviral vectoring system pseudotyped with the vesicular stomatitis virus (VSV) envelope and which contained the green fluorescent protein (GFP) gene as a marker was used (Figure 1). Recombinant lentiviruses were produced as described by Naldini et al. The cytomegalovirus (CMV) immediate-early gene promoter directed expression of eGFP in the plasmid pHR'-CMV-eGFP. Stocks of virus were generated as follows. Human kidney 293T cells (5x10⁶) were plated on 10 cm plates, and were cotransfected the following day with 10 ug of pCMVΔR8.91 (packaging function plasmid), 10 ug of pHR'-CMV-eGFP (marker gene plasmid), and 2 ug of pMD.G (the VSV-G envelope containing plasmid) by calcium phosphate precipitation in D10 growth medium (high glucose DMEM with 10% fetal bovine serum) and antibiotics. After 12-16 h at 37°C , the medium was removed and fresh D10 growth medium was added. Cells were cultured for an additional 10 h. Fresh D10 medium containing 10mM sodium butyrate and 20mM Hepes buffer was added to the cells and the cells were cultured for another 12 h. This medium was replaced with new D10 medium containing 20mM Hepes buffer, and after 12 h the virus-containing medium was collected. Fresh medium was added and the supernatant was collected every 24 h for the following 4 days. The viral supernatant was stored at -80°C immediately after collection.

Viral stock were concentrated by ultracentrifugation of the supernatant (19,000 rpm, Beckman SW28 rotor) for 140 min at room temperature and the resulting viral pellets were resuspended in 1-3 ml of phosphate-buffered saline. Aliquoted viral stocks were titered with 293 cells and the remaining samples were stored at -80°C.

All lentiviral vector supernatants were assayed for the presence of replication competent retrovirus (RCR) by infection of phytohemagglutinin-stimulated human peripheral blood mononuclear cells, with subsequent analysis of the culture medium for p24 gag by ELISA. RCR was not detected in any of the viral supernatants produced.

### EXAMPLE 3

### Lentivirus Vector Transduction

Supernatants containing 2 x 10⁶ replication-deficient lentiviral particles/ml were generated by the transfection of 293T cells with the lentivirus vector described above. Cells were cultured with the viral particles for 24 hours and then recovered in normal media for four days prior to the determination of GFP expression by fluorescent-activated cell sorting (Figures 2-3).

Transduction efficiency was measured as a function of multiplicity of infection with MOIs ranging from 1 to 1000. Results of *in vitro* transduction of a number of human cell lines demonstrate a positive correlation between MOI and transduction efficiency as more cells were transduced with increasing number of lentiviral particles (Figure 2).

The ability of the lentiviral vector to transduce non-dividing cells was examined. Human retinal pigment epithelial cells were transduced by lentiviral or murine leukemia viral vectors. Cells were mitotically inactive (confluent) or mitotically active (growing) at the time of exposure to vector. Results shown in Figure 4 demonstrate a superior ability of lentiviral vectors over other retroviral vectors to transduce non-dividing cells. The lentiviral vector was also highly efficient in transducing human fetal cells as compared with non-lentiviral retroviral vector (Figure 6).

To determine the duration of eGFP transgene expression, cells transduced by the lentiviral vector were tested over a period of 120 days. Results of Southern Blot analysis on clonal populations of transduced cells indicate that the lentiviral-eGFP vector was integrated into the host genome (Figure 5B). Expression of the integrated eGFP transgene was stable over 120 days and confer no selective advantage for or against the transduced cells (Figure 5A).

### EXAMPLE 4

### Corneal Transdcution in situ

Human corneal buttons obtained at the time of corneal transplant surgery were used to demonstrate the ability of lentiviral vectors to transduce these mitotically inactive cells with the marker gene enhanced green fluorescence protein gene (Figure 7). Endothelial cells attached to Descemet's membrane were peeled away from the transduced corneal tissue, and examined by light and fluorescent microscopy. The corneal endothelium was positive for eGFP, indicating that efficient gene transfer and expression were attained (Figure 7B). Efficient in situ transduction and eGFP expression in the epithelial layer was also observed (Figure 7C).

In conclusion, these results indicate that a replication-defective lentiviral vector is able to transfer efficiently transgene to human corneal endothelial and epithelial cells *in situ,* and achieve long-term transgene expression. This vector could be useful in the treatment of corneal endothelial or epithelial disorders and can be applied to modify the genetic makeup of a donor cornea tissue *ex vivo* before transplantation in such a way as to modulate permanently the process of allograft rejection.

### EXAMPLE 5

### Growth Suppressor Therapy For Ocular Proliferative Disease

Human peripherin gene was used as one example of therapeutic gene. Genetic deficiency of peripherin gene in humans is known to result in a wide variety of disabling phenotypes. Normal human retinal or retinal pigment epithelial (RPE) tissue surgically excised at the time of enucleation for retinoblastoma was exposed to lentiviral vectors which either lacked a therapeutic gene or contained the human peripherin gene. Results in Figure 8 demonstrate that the peripherin gene was efficiently transferred to human retinal tissue by the lentiviral vector.

As an another example of therapeutic gene transfer, the constitutively active form of the retinoblastoma gene (CA-rb) was used. The lentiviral vector disclosed herein mediated efficient transfer of the constitutively active form of the retinoblastoma gene (Figure 9). The transferred CA-rb gene exhibited dose-dependent inhibitory effects on the proliferation of human retinal and choroidal cells (Figure 10) and human lens epithelial cells (Figure 11).

The constitutively active form of the retinoblastoma gene transferred by the lentiviral vector also inhibited intraocular cellular proliferation *in vivo.* Two models of intraocular proliferative disease (proliferative vitreoretinopathy and post-lens extraction posterior capsular opacification) were tested *in vivo.* Proliferative vitreoretinopathy was induced in three sets of rabbits (Figure 12). One set was not treated, one set was treated with lentiviral vectors lacking the CA-rb gene and the last set was treated with intravitreally-delivered lentiviral CA-rb. Proliferative vitreoretinopathy and retinal detachment was noted in the first two sets at high frequency (>90%), whereas the fraction of animals that went on to retinal detachment was significantly lower in the set treated with CA-rb (26%).

Results shown in Figure 13 demonstrate *in vivo* inhibitory effect of lentiviral CA-rb on the process of post-lens extraction posterior capsular opacification. Three sets of rabbits underwent standard phacoemulsfication to remove the native crystalline lens. The first set (group 1) was subsequently treated with nothing and the second two sets were treated with either empty lentiviral constructs (no therapeutic gene, group 2) or with lentiviral CA-rb (group 3) delivered into the intact lens capsular bag at the time of closure of the cataract wound. Animals were serially examined for the presence of posterior capsular opacification. The presence of opacification was graded on a 1 to 5 scale where 1 represented no opacification and 5 represented opacification severe enough to preclude visualization of the retina with indirect binocular ophthalmoscopy. There were no statistically different results obtained between groups 1 and 2 (no treatment and empty vector), whereas a striking inhibitory effect of lentiviral CA-rb on the development of posterior capsule opacification was observed. By day 28, control animals had an average opacification score of 4.4 while animals treated with lentiviral CA-rb had an average opacification score of 2.1.

### EXAMPLE 6

### "Two Gene" Lentiviral Vector

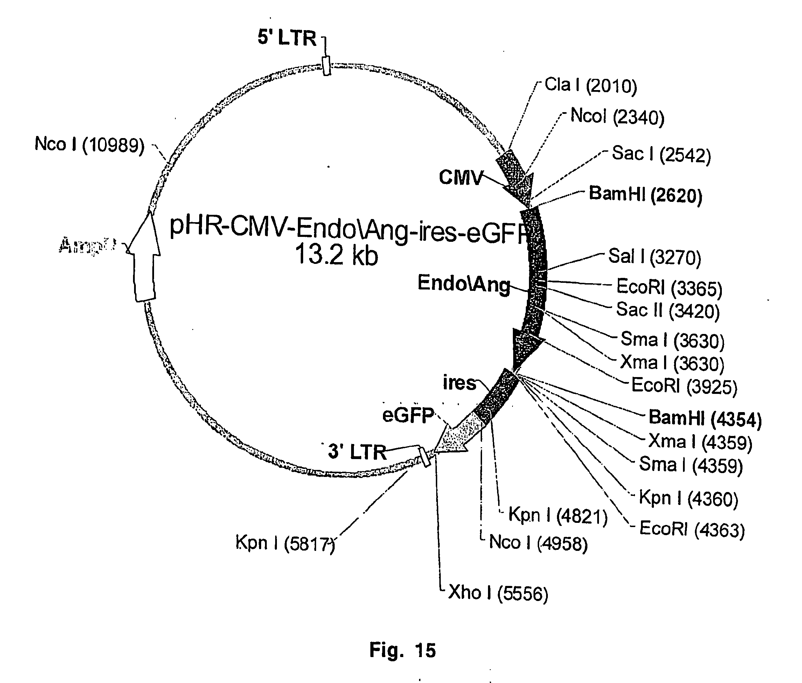

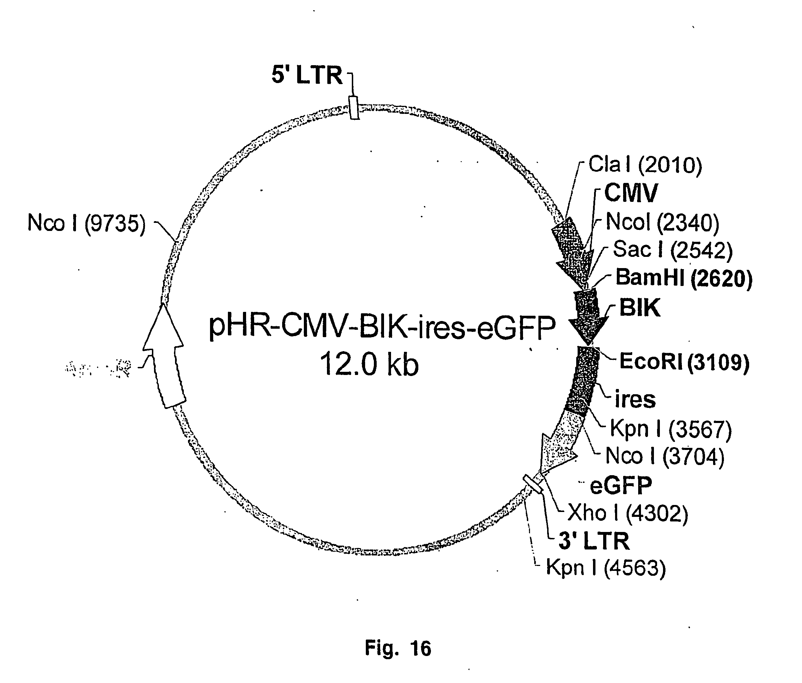

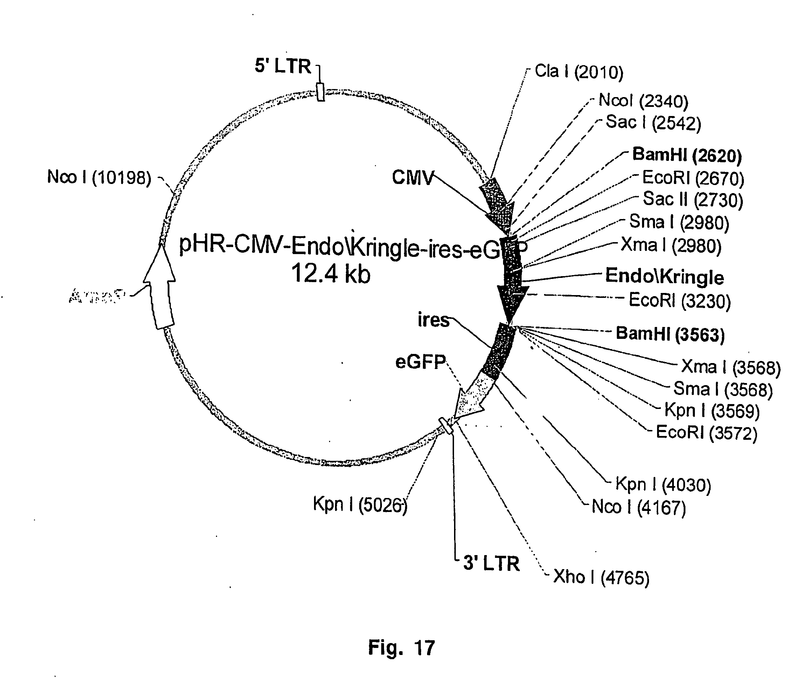

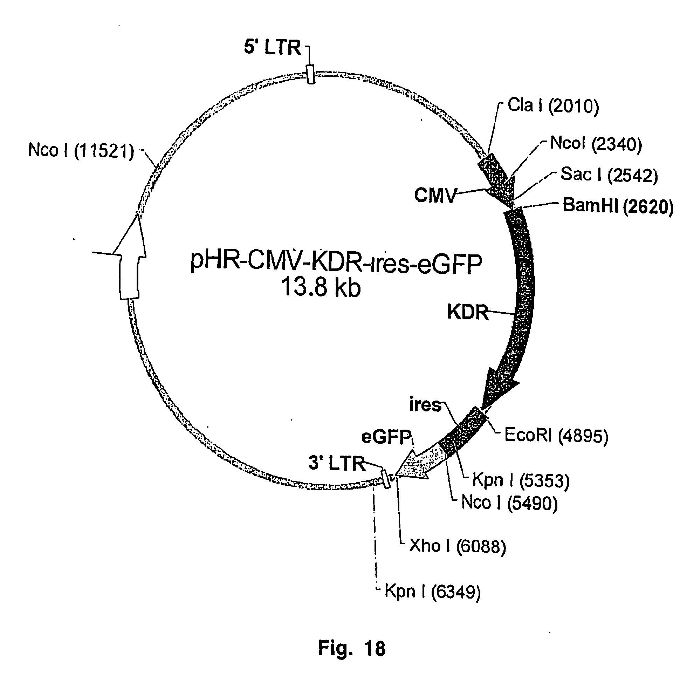

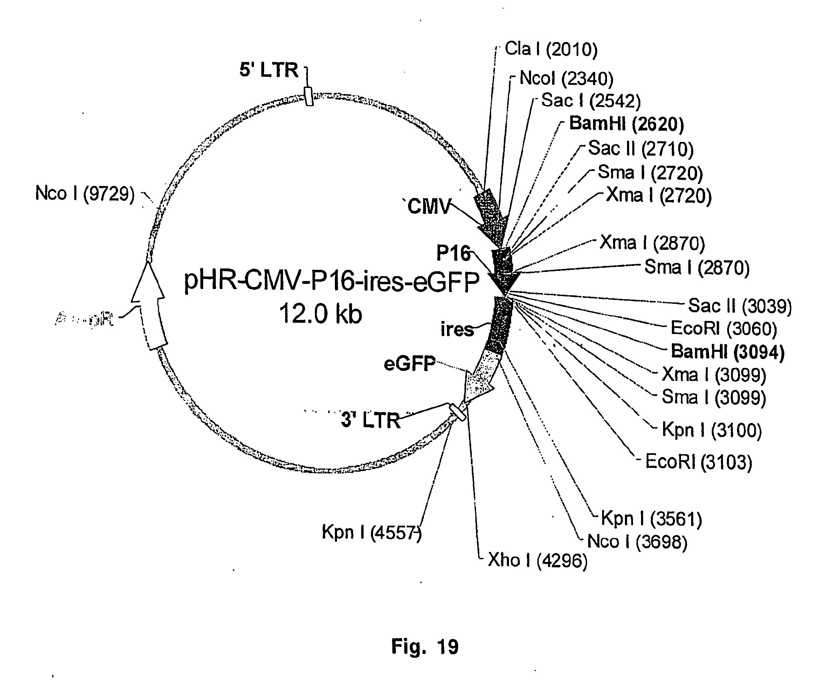

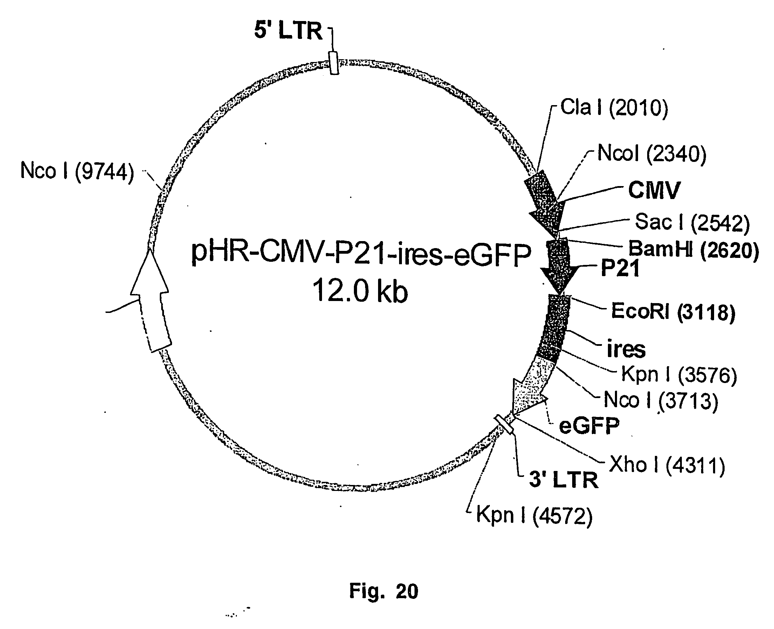

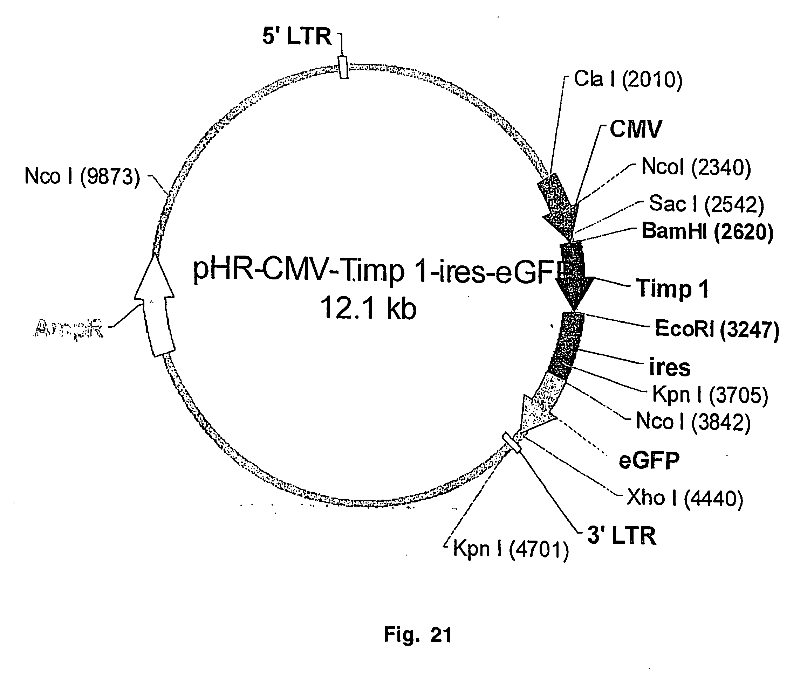

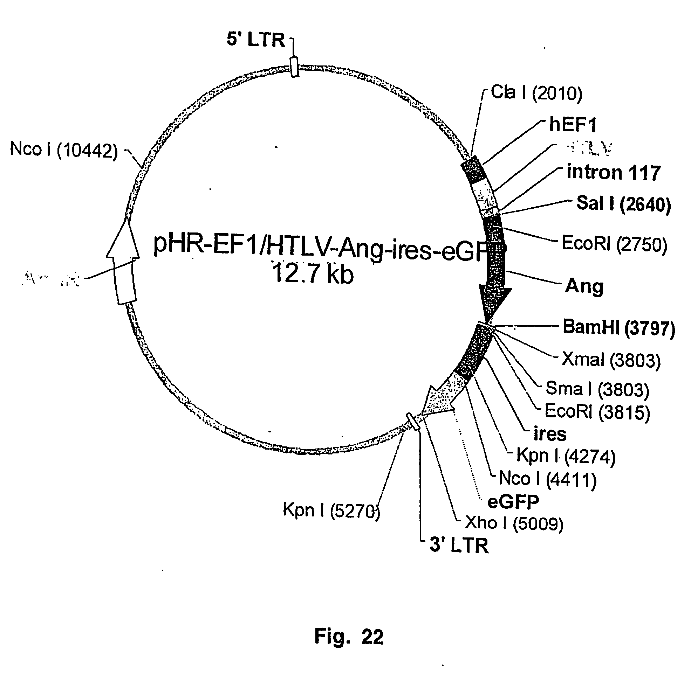

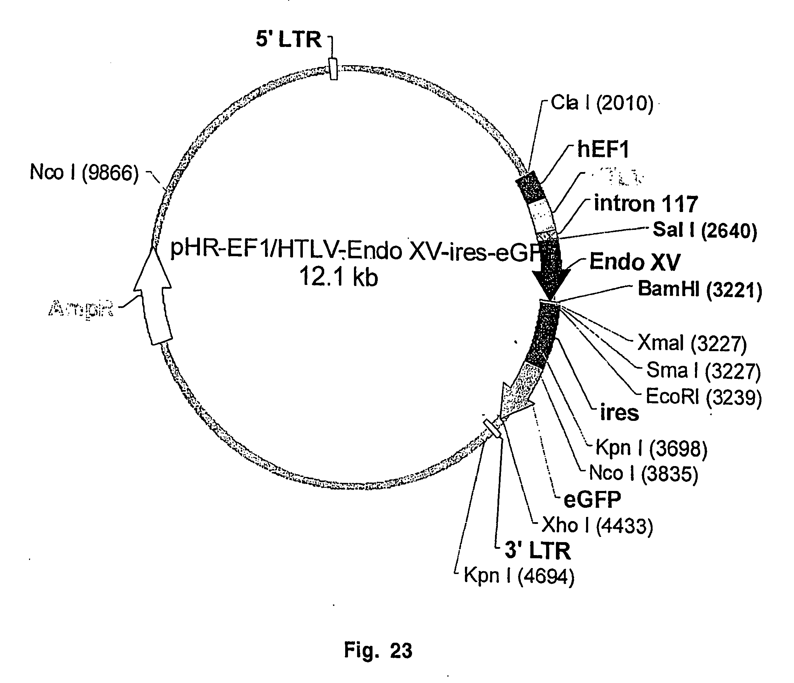

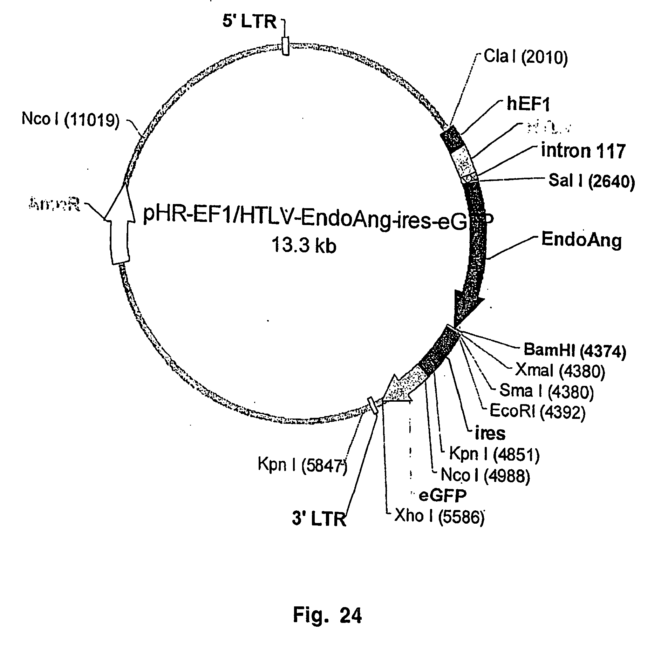

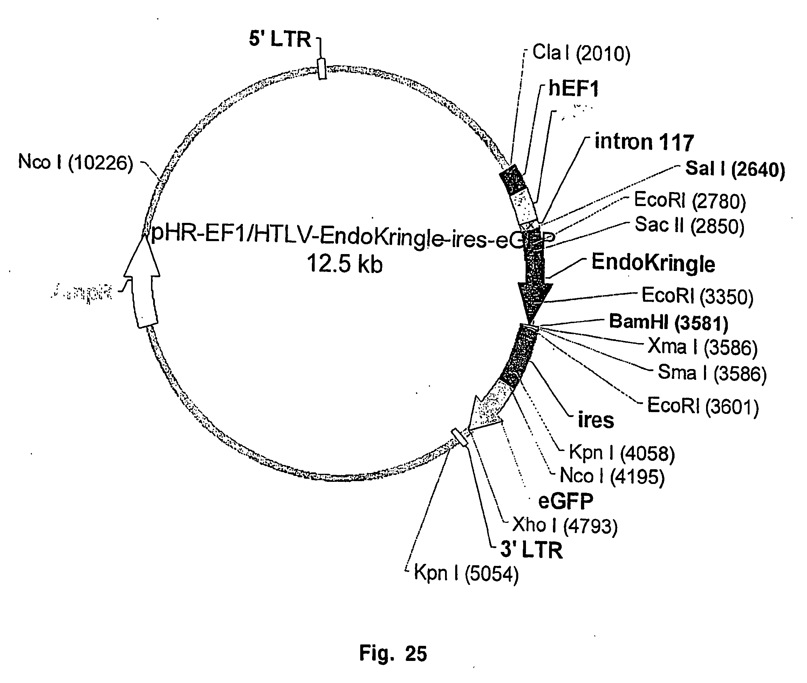

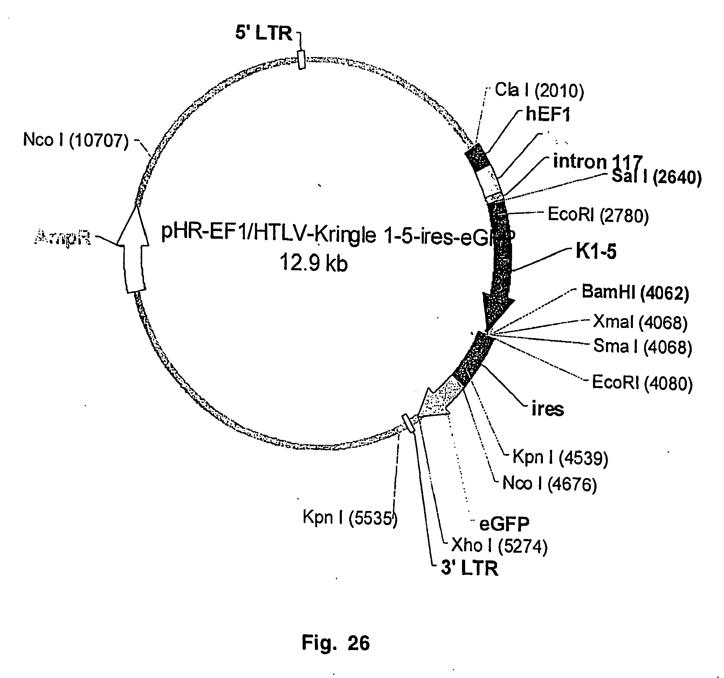

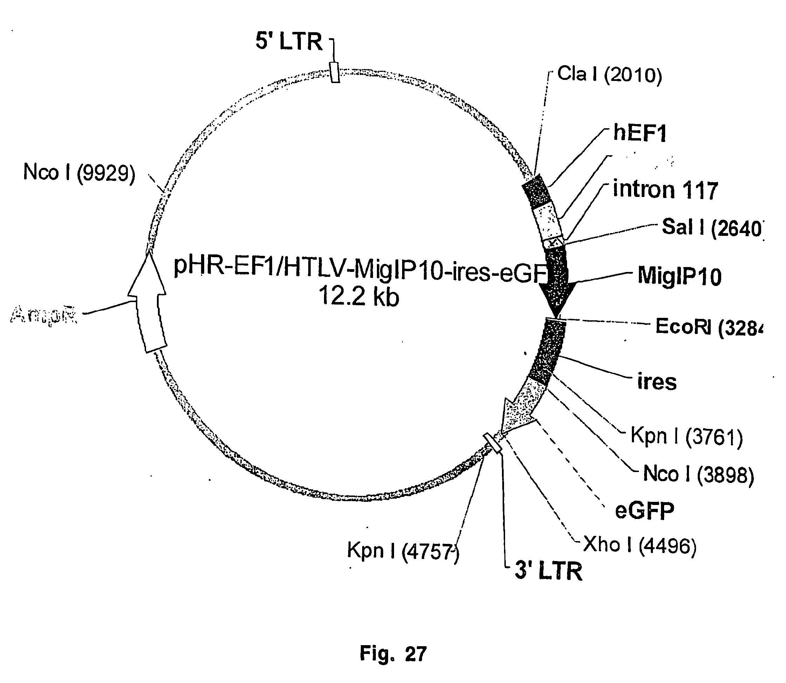

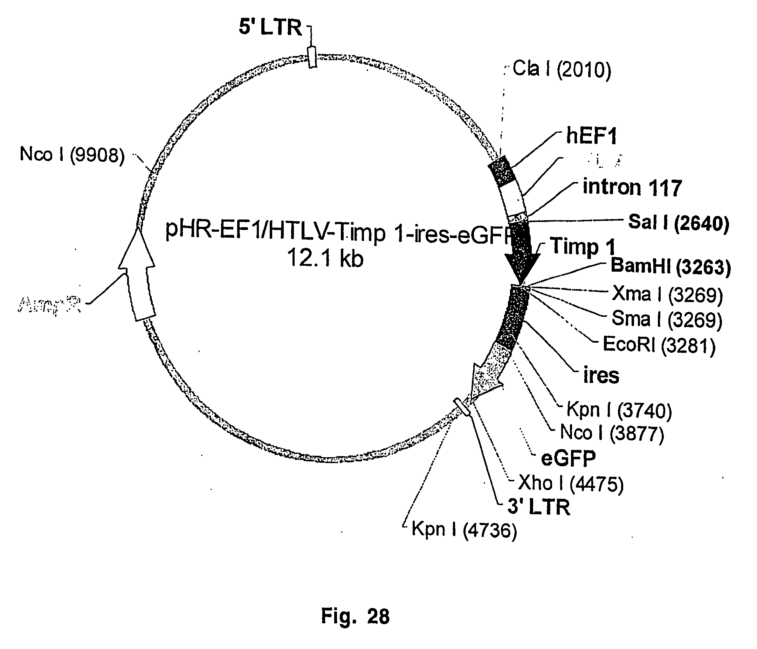

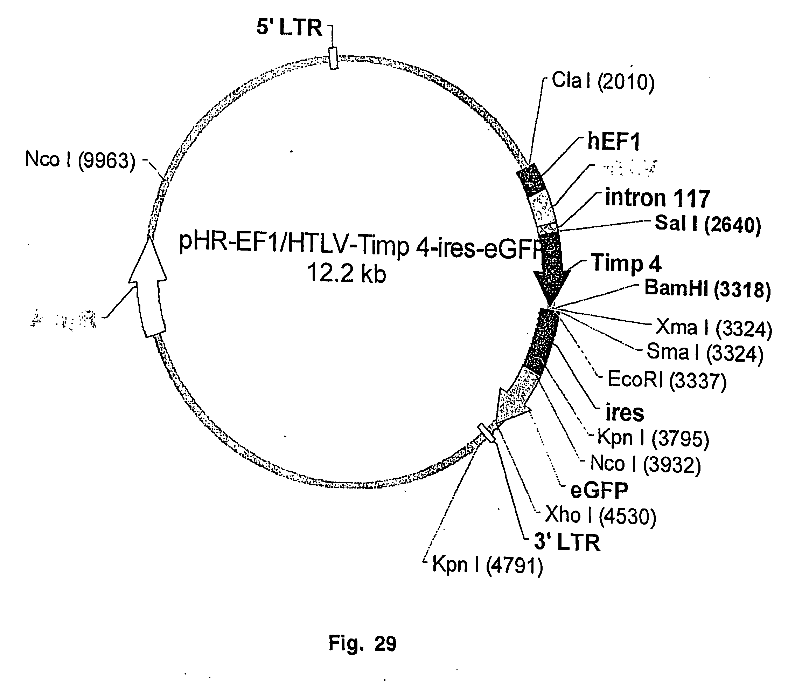

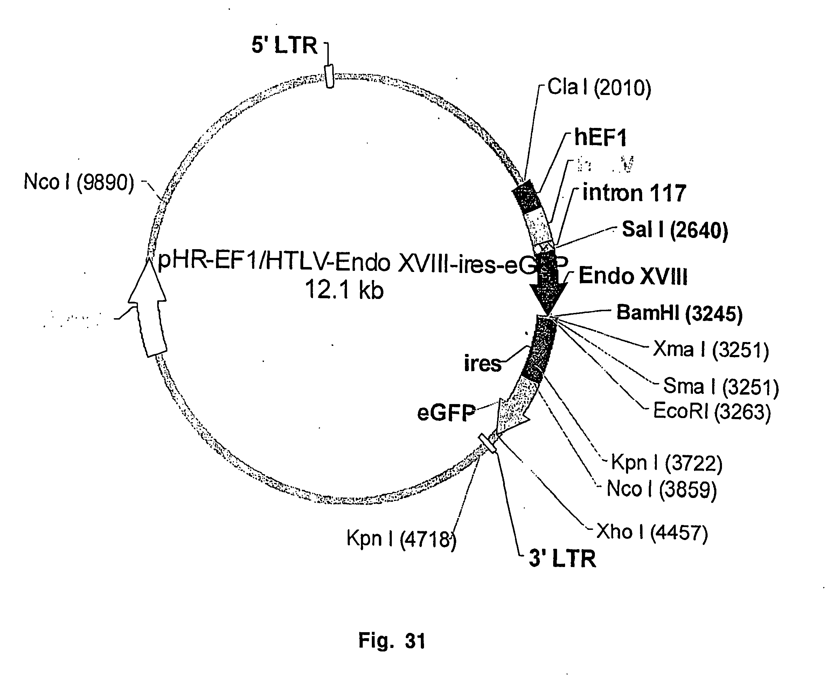

A new lentiviral vector that incorporated an IRES (internal ribosome entry site) element between two cloning sites was constructed. The IRES element allows mRNA-ribosome binding and protein synthesis. This backbone can accommodate two different expressible genes. A single message is produced in transduced cells; however, because of the IRES element, this message is functionally bi-cistronic and can drive the synthesis of two different proteins. In this fashion each of the potentially therapeutic genes discussed above can be linked to a marker gene (e.g. the enhanced green fluorescent gene - eGFP gene) so that transduced cells will simultaneously be marked and able to express the therapeutic gene of interest. Marked cells can easily be isolated *in vitro* and observed *in vivo.* Genetic maps for a number of lentiviral vectors carrying various therapeutic gene are shown in Figures 15-31. Since the level of ordinary skill of an average scientist in the areas of genetic engineering and cloning has increased substantially in recent years, a person having ordinary skill in this art would readily be able to construct lentiviral vectors containing other therapeutic genes of interest.

### EXAMPLE 7

### Anti-Neovascularization Gene Therapy

Naive cells (cells known to not express the therapeutic gene) were exposed to the aforementioned lentiviral vectors for 24 hours. Two days following this exposure, RNA was isolated from these cells and was tested for transgene expression by reverse-transcriptase assisted polymerase chain reaction (RT-PCR). Figure 32 shows a positive RT-PCR product for the endostatin-18/angiostatin fusion gene from mRNA isolated from human dermal microvascular endothelial cells.

Following the demonstration *of in vitro* lentiviral-mediated gene transfer as shown above, the ability to inhibit neovascularization *in vivo* was then examined. Neovascularization was induced in rabbit corneal tissues in the following fashion:

### Creation Of A Corneal Intrastromal Micropocket And Insertion Of Nylon Mesh Impregnated With Lentivirus

Rabbits underwent general anesthesia with Isoflourane (4 L/Min) and Oxygen (2 L/Min) by masking. One drop of Proparacaine was placed in the fornix for topical anesthesia. The Isoflourane was reduced to 2.5 L/Min. Betadine was placed in the fornix for 30 sec. and rinsed out with BSS (balanced saline solution, Alcon Inc). A lid speculum was placed in the eye. A 2.8 mm microkeratome was used to enter the corneal stroma at 12 o'clock. This intrastromal incision was developed into a 5 x 5 mm intrastromal pocket with a McPherson forceps and Iris Sweep instrument by sweeping back-and-forth. The 12 o'clock incision was opened up on either side so that the opening was 4.5 mm with Vannas scissors. A 4 x 4 mm Amersham hybridization nylon mesh (Amersham Bioscientist RPN 2519) impregenated with 10 µL of lentivirus was inserted into the pre-formed pocket. A drop of tobramycin was placed on the cornea. Isoflourane was discontinued and nasal oxygen was increased to 4 L/Min. In this fashion, rabbits were successfully brought out of general anesthesia after 20 minutes and returned to their cages with normal vital functions. Rabbits received 0.2cc of buprenex (.3mg/cc) SQ bid for two days for analgesia. Rabbits also received one drop of atropine and one drop of tobramycin for two days for post-op cycloplegia and antibiotic care. On the first post-operative day each rabbit received a drop of topical proparacaine for anesthesia and the nylon mesh was removed from the corneal intrastromal pocket with a 12 forces. Post surgical pain control and care was monitored daily for two weeks.

### Alkali Induced Neovascularization

Two weeks after initial surgery, corneas were exposed to 6mm Whatman #3 filter disks saturated with 20 µl of 1.0M NaOH for 1 minute. All corneas were then copiously washed with BSS. Rabbits received one drop of atropine and one drop of tobramycin for two days for post-op cycloplegia and antibiotic care. Digital photo-documentation was carried out to record the neovascular response. The neovascular response was measured by slit-lamp examination noting the clock hours and the length of vessels on post-trauma day 1, 3, 5, 7, and 10. Neovascularization was quantified by calculating the area of vessel growth as described below.

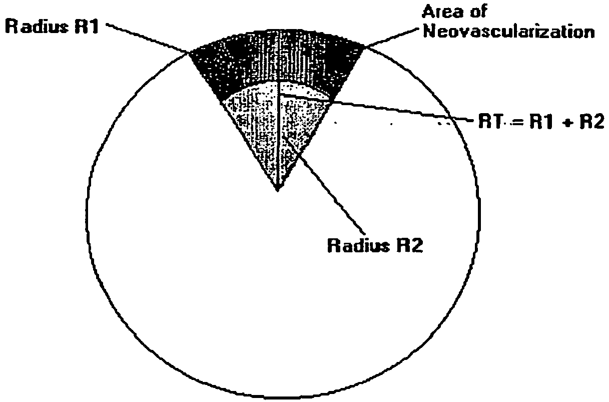

For a standardized method of evaluation for corneal neovascularization, the following protocol and formula were devised to record and compare the neovascularization after the alkali burn. The formula for the area of neovascularization is derived by calculating the area of the larger sector bounded by radius **RT** and subtracting the smaller sector bounded by radius **R2.** The area of the larger sector bounded by radius **RT is** the number of clock hours divided by 12 and multiplied by π**R T**². The area of the smaller sector bounded by radius **R2** is the number of clock hours divided by 12 and multiplied by π(**R 2**)². The resulting area derived from the subtraction of the two sectors would be the area of neovascularization.

Confocal microscopy was performed to document the expression of enhanced green fluorescent protein, the marker gene included in the lentiviral bicistronic message. Figure 33 shows photomicrographs demonstrating the presence of eGFP within the corneal micropocket in animals treated with the lentiviral vector.

To demonstrate an inhibitory effect on neovascularization, neovascularization was induced in animals as described above. After treatment with lentiviral vector containing a Mig/IP10 fusion gene, an inhibitory effect were observed (Figure 34). As shown in Table 1, significant reduction of neovascularization was observed in animals treated with the Mig/IP10 fusion gene or a Kringle 1-5 gene transferred by the lentiviral vectors.

**TABLE 1**

| Inhibiting of Neovascularizaiton After Lentiviral Gene Transfer | | |
|---|---|---|
| GENE | mm² of neovascularization Treated animals | mm²of neovascularization Untreated animals |
| Mig/IP10 fusion gene | 57.0 mm² | 132.2 mm² |
| Kringle 1-5 | 0.9 mm² | 17.0 mm² |

The following references were cited herein:
Naldini et al., (1996) Science 272: 263-267.
Miyoshi et al., (1997) Proc. Natl. Acad. Sci. USA 94: 10319-10323.

Any patents or publications mentioned in this specification are indicative of the levels of those skilled in the art to which the invention pertains. Further, these patents and publications are incorporated by reference herein to the same extent as if each individual publication was specifically and individually indicated to be incorporated by reference.

One skilled in the art will appreciate readily that the present invention is well adapted to carry out the objects and obtain the ends and advantages mentioned, as well as those objects, ends and advantages inherent herein. The present examples, along with the methods, procedures, treatments, molecules, and specific compounds described herein are presently representative of preferred embodiments,

## Claims

1. A recombinant lentiviral vector comprising a first therapeutic gene that reduces or inhibits ocular angiogenesis for use in the treatment of age-related macular degeneration in an individual, wherein said first therapeutic gene is selected from the group consisting of endostatin, angiostatin, endostatin XVIII, endostatin XV, the C-terminal hemopexin domain of matrix metalloproteinase-2, the kringle 5 domain of human plasminogen, the monokine-induced by interferon-gamma (Mig), the interferon-alpha inducible protein 10 (IP10), soluble FLT-1 (fms-like tyrosine kinase 1 receptor), and kinase insert domain receptor (KDR).

2. The lentiviral vector of claim 1, wherein said first therapeutic gene encodes endostatin.

3. The lentiviral vector of claim 1, wherein said first therapeutic gene encodes angiostatin.

4. The lentiviral vector of claim 1, wherein said first therapeutic gene encodes endostatin XVIII.

5. The lentiviral vector of claim 1, wherein said first therapeutic gene encodes endostatin XV.

6. The lentiviral vector of claim 1, wherein said first therapeutic gene encodes the C-terminal hemopexin domain of matrix metalloproteinase-2.

7. The lentiviral vector of claim 1, wherein said first therapeutic gene encodes the kringle 5 domain of human plasminogen.

8. The lentiviral vector of claim 1, wherein said first therapeutic gene encodes Mig.

9. The lentiviral vector of claim 1, wherein said first therapeutic gene encodes IP10.

10. The lentiviral vector of claim 1, wherein said first therapeutic gene encodes soluble FLT-1.

11. The lentiviral vector of claim 1, wherein said first therapeutic gene encodes KDR.

12. The lentiviral vector of claim 1, further comprising a second therapeutic gene that inhibits angiogenesis, wherein said second therapeutic gene is selected from the group consisting of endostatin, angiostatin, endostatin XVIII, endostatin XV, the C-terminal hemopexin domain of matrix metalloproteinase-2, the kringle 5 domain of human plasminogen, the monokine-induced by interferon-gamma (Mig), the interferon-alpha inducible protein 10 (IP10), soluble FLT-1 (fms-like tyrosine kinase 1 receptor), and kinase insert domain receptor (KDR), and wherein the lentiviral vector encodes a fusion protein comprising a first amino acid sequence encoded by the first therapeutic gene and a second amino acid sequence encoded by the second therapeutic gene.

13. The lentiviral vector of claim 1, further comprising a second therapeutic gene that inhibits angiogenesis, wherein said second therapeutic gene is selected from the group consisting of endostatin, angiostatin, endostatin XVIII, endostatin XV, the C-terminal hemopexin domain of matrix metalloproteinase-2, the kringle 5 domain of human plasminogen, the monokine-induced by interferon-gamma (Mig), the interferon-alpha inducible protein 10 (IP10), soluble FLT-1 (fms-like tyrosine kinase 1 receptor), and kinase insert domain receptor (KDR), and wherein the lentiviral vector comprises an IRES (internal ribosome entry site) element between said first therapeutic gene and said second therapeutic gene so that a first amino acid sequence encoded by the first therapeutic gene and a second amino acid sequence encoded by the second therapeutic gene are produced from a single transcript.

14. The lentiviral vector of claim 12, further comprising a linker between the first amino acid sequence and the second amino acid sequence.

15. The lentiviral vector of claim 12, wherein the lentiviral vector encodes a fusion protein of endostatin XVIII and angiostatin.

16. The lentiviral vector of claim 12, wherein the lentiviral vector encodes a fusion protein of endostatin XVIII and the kringle 5 domain of human plasminogen.

17. The lentiviral vector of claim 12, wherein the lentiviral vector encodes a fusion protein of Mig and IP10.

18. Use of a lentiviral vector comprising a therapeutic gene that reduces or inhibits ocular angiogenesis in the manufacture of a medicament for inhibiting neovascularization in an individual having age-related macular degeneration, wherein said therapeutic gene is selected from the group consisting of endostatin, angiostatin, endostatin XVIII, endostatin XV, the C-terminal hemopexin domain of matrix metalloproteinase-2, the kringle 5 domain of human plasminogen, a fusion protein of endostatin and angiostatin, a fusion protein of endostatin and the kringle-5 domain of human plasminogen, the monokine-induced by interferon-gamma (Mig), the interferon-alpha inducible protein 10 (IP10), a fusion protein of Mig and IP10, soluble FLT-1 (fms-like tyrosine kinase 1 receptor), and kinase insert domain receptor (KDR).

19. Use according to claim 18, wherein the lentiviral vector is a lentiviral vector as set forth in any of claims 1-17.

20. Use according to claim 18, wherein said lentiviral vector is administered in a dosage of from about 10⁶ to 10⁹ transducing particles into the capsular, vitreal or sub-retinal space.

21. A method for transducing ex vivo corneal tissue with a therapeutic gene, which comprises exposing a corneal cell which forms part of a corneal tissue to a recombinant lentiviral vector comprising said therapeutic gene operatively coupled to a promoter known to be active in corneal cells, wherein the therapeutic gene is selected from the group consisting of endostatin, angiostatin, endostatin XVIII, endostatin XV, the C-terminal hemopexin domain of matrix metalloproteinase-2, the kringle 5 domain of human plasminogen, the monokine-induced by interferon-gamma (Mig), the interferon-alpha inducible protein 10 (IP10), soluble FLT-1 (fms-like tyrosine kinase 1 receptor), and kinase insert domain receptor (KDR).

22. A corneal tissue comprising cells transduced with a recombinant lentiviral vector, wherein the recombinant lentiviral vector comprises a first therapeutic gene operatively coupled to a promoter known to be active in corneal cells, and wherein the therapeutic gene is selected from the group consisting of endostatin, angiostatin, endostatin XVIII, endostatin XV, the C-terminal hemopexin domain of matrix metalloproteinase-2, the kringle 5 domain of human plasminogen, the monokine-induced by interferon-gamma (Mig), the interferon-alpha inducible protein 10 (IP10), soluble FLT-1 (fms-like tyrosine kinase 1 receptor), and kinase insert domain receptor (KDR).

23. The corneal tissue of claim 22, wherein the cells are transduced with a lentiviral vector as set forth in any of claims 1-17.

24. The corneal tissue of claim 22, wherein the corneal tissue is a human corneal tissue.

25. A corneal tissue as set forth in any of claims 22-24 for use in the treatment of corneal neovascularization in an individual.

26. The corneal tissue of claim 25, wherein the corneal tissue is transduced *in situ.*

## Patentansprüche

1. Rekombinanter Lentivirus-Vektor, umfassend ein erstes therapeutisches Gen, welches die okulare Angiogenese vermindert oder inhibiert, zur Verwendung bei der Behandlung von altersbedingter Makuladegeneration bei einem Individuum, wobei das erste therapeutische Gen aus der aus Endostatin, Angiostatin, Endostatin XVIII, Endostatin XV, der C-terminalen Hämopexindomäne der Matrixmetalloproteinase-2, der Kringle-5-Domäne von humanem Plasminogen, dem durch Interferon-gamma induzierten Monokin (Mig), dem durch Interferon-alpha induzierbaren Protein 10 (IP10), löslichem FLT-1 (dem Rezeptor für Fms-Like Tyrosinkinase 1) und dem Kinase Insert Domain Receptor (KDR) bestehenden Gruppe ausgewählt ist.

2. Lentivirus-Vektor nach Anspruch 1, wobei das erste therapeutische Gen für Endostatin codiert.

3. Lentivirus-Vektor nach Anspruch 1, wobei das erste therapeutische Gen für Angiostatin codiert.

4. Lentivirus-Vektor nach Anspruch 1, wobei das erste therapeutische Gen für Endostatin XVIII codiert.

5. Lentivirus-Vektor nach Anspruch 1, wobei das erste therapeutische Gen für Endostatin XV codiert.

6. Lentivirus-Vektor nach Anspruch 1, wobei das erste therapeutische Gen für die C-terminale Hämopexindomäne von Matrixmetalloproteinase-2 codiert.

7. Lentivirus-Vektor nach Anspruch 1, wobei das erste therapeutische Gen für die Kringle-5-Domäne von humanem Plasminogen codiert.

8. Lentivirus-Vektor nach Anspruch 1, wobei das erste therapeutische Gen für Mig codiert.

9. Lentivirus-Vektor nach Anspruch 1, wobei das erste therapeutische Gen für IP10 codiert.

10. Lentivirus-Vektor nach Anspruch 1, wobei das erste therapeutische Gen für löslichen FLT-1 codiert.

11. Lentivirus-Vektor nach Anspruch 1, wobei das erste therapeutische Gen für KDR codiert.

12. Lentivirus-Vektor nach Anspruch 1, weiterhin umfassend ein zweites therapeutisches Gen, welches Angiogenese inhibiert, wobei das zweite therapeutische Gen aus der aus Endostatin, Angiostatin, Endostatin XVIII, Endostatin XV, der C-terminalen Hämopexindomäne der Matrixmetalloproteinase-2, der Kringle-5-Domäne von humanem Plasminogen, dem durch Interferon-gamma induzierten Monokin (Mig), dem durch Interferon-alpha induzierbaren Protein 10 (IP10), löslichem FLT-1 (dem Rezeptor für Fms-Like Tyrosinkinase 1) und dem Kinase Insert Domain Receptor (KDR) bestehenden Gruppe ausgewählt ist, und wobei der Lentivirus-Vektor für ein Fusionsprotein codiert, welches eine erste, von dem ersten therapeutischen Gen codierte Aminosäuresequenz und eine zweite, von dem zweiten therapeutischen Gen codierte Aminosäuresequenz umfasst.

13. Lentivirus-Vektor nach Anspruch 1, weiterhin umfassend ein zweites therapeutisches Gen, welches Angiogenese inhibiert, wobei das zweite therapeutische Gen aus der aus Endostatin, Angiostatin, Endostatin XVIII, Endostatin XV, der C-terminalen Hämopexindomäne der Matrixmetalloproteinase-2, der Kringle-5-Domäne von humanem Plasminogen, dem durch Interferon-gamma induzierten Monokin (Mig), dem durch Interferon-alpha induzierbaren Protein 10 (IP10), löslichem FLT-1 (dem Rezeptor für Fms-Like Tyrosinkinase 1) und dem Kinase Insert Domain Receptor (KDR) bestehenden Gruppe ausgewählt ist, und wobei der Lentivirus-Vektor ein IRES (Internal Robosome Entry Site)- Element zwischen dem ersten therapeutischen Gen und dem zweiten therapeutischen Gen umfasst, so dass von einem einzelnen Transkript eine erste, von dem ersten therapeutischen Gen codierte Aminosäuresequenz und eine zweite, von dem zweiten therapeutischen Gen codierte Aminosäuresequenz produziert werden.

14. Lentivirus-Vektor nach Anspruch 12, weiterhin umfassend einen Linker zwischen der ersten Aminosäuresequenz und der zweiten Aminosäuresequenz.

15. Lentivirus-Vektor nach Anspruch 12, wobei der Lentivirus-Vektor für ein Fusionsprotein von Endostatin XVIII und Angiostatin codiert.

16. Lentivirus-Vektor nach Anspruch 12, wobei der Lentivirus-Vektor für ein Fusionsprotein von Endostatin XVIII und der Kringle-5-Domäne von humanem Plasminogen codiert.

17. Lentivirus-Vektor nach Anspruch 12, wobei der Lentivirus-Vektor für ein Fusionsprotein von Mig und IP10 codiert.

18. Verwendung eines Lentivirus-Vektors, umfassend ein therapeutisches Gen, welches okulare Angiogenese vermindert oder inhibiert, zur Herstellung eines Medikaments zur Inhibierung der Gefäßneubildung bei einem Individuum mit altersbedingter Makuladegeneration, wobei das therapeutische Gen aus der aus Endostatin, Angiostatin, Endostatin XVIII, Endostatin XV, der C-terminalen Hämopexindomäne der Matrixmetalloproteinase-2, der Kringle-5-Domäne von humanem Plasminogen, einem Fusionsprotein von Endostatin und Angiostatin, einem Fusionsprotein von Endostatin und der Kringle-5-Domäne von humanem Plasminogen, dem durch Interferon-gamma induzierten Monokin (Mig), dem durch Interferon-alpha induzierbaren Protein 10 (IP10), einem Fusionsprotein von Mig und IP10, löslichem FLT-1 (dem Rezeptor für Fms-Like Tyrosinkinase 1) und dem Kinase Insert Domain Receptor (KDR) bestehenden Gruppe ausgewählt ist.

19. Verwendung nach Anspruch 18, wobei es sich bei dem Lentivirus-Vektor um einen wie in einem der Ansprüche 1-17 ausgeführten Lentivirus-Vektor handelt.

20. Verwendung nach Anspruch 18, wobei der Lentivirus-Vektor in einer Dosierung von etwa 10⁶ bis etwa 10⁹ transduzierenden Partikeln in den Kapselraum, den Glaskörperraum oder den unter der Retina liegenden Raum verabreicht wird.

21. Verfahren zu einer ex-vivo-Transduktion von Hornhautgewebe mit einem therapeutischen Gen, bei dem man eine Hornhautzelle, die Teil eines Hornhautgewebes ist, der Einwirkung eines rekombinanten Lentivirus-Vektors, welcher das therapeutische Gen funktionell an einen Promotor, von dem bekannt ist, dass er in Hornhautzellen aktiv ist, gekoppelt enthält, aussetzt, wobei das therapeutische Gen aus der aus Endostatin, Angiostatin, Endostatin XVIII, Endostatin XV, der C-terminalen Hämopexindomäne der Matrixmetalloproteinase-2, der Kringle-5-Domäne von humanem Plasminogen, dem durch Interferon-gamma induzierten Monokin (Mig), dem durch Interferon-alpha induzierbaren Protein 10 (IP10), löslichem FLT-1 (dem Rezeptor für Fms-Like Tyrosinkinase 1) und dem Kinase Insert Domain Receptor (KDR) bestehenden Gruppe ausgewählt ist.

22. Hornhautgewebe, umfassend mit einem rekombinanten Lentivirus-Vektor transduzierte Zellen, wobei der rekombinante Lentivirus-Vektor ein erstes therapeutisches Gen umfasst, welches funktionell an einen Promotor gekoppelt ist, von dem bekannt ist, dass er in Hornhautzellen aktiv ist, und wobei das therapeutische Gen aus der aus Endostatin, Angiostatin, Endostatin XVIII, Endostatin XV, der C-terminalen Hämopexindomäne der Matrixmetalloproteinase-2, der Kringle-5-Domäne von humanem Plasminogen, dem durch Interferon-gamma induzierten Monokin (Mig), dem durch Interferon-alpha induzierbaren Protein 10 (IP10), löslichem FLT-1 (dem Rezeptor für Fms-Like Tyrosinkinase 1) und dem Kinase Insert Domain Receptor (KDR) bestehenden Gruppe ausgewählt ist.

23. Hornhautgewebe nach Anspruch 22, wobei die Zellen mit einem wie in einem der Ansprüche 1-17 ausgeführten Lentivirus-Vektor transduziert sind.

24. Hornhautgewebe nach Anspruch 22, wobei es sich bei dem Hornhautgewebe um humanes Hornhautgewebe handelt.

25. Hornhautgewebe, ausgeführt wie in einem der Ansprüche 22-24, zur Verwendung bei der Behandlung einer Gefäßneubildung in der Hornhaut eines Individuums.

26. Hornhautgewebe nach Anspruch 25, wobei das Hornhautgewebe *in situ* transduziert wird.

## Revendications

1. Vecteur lentiviral recombinant comprenant un premier gène thérapeutique qui réduit ou inhibe l'angiogenèse oculaire, pour utilisation dans le traitement de la dégénérescence maculaire liée à l'âge chez un individu, dans lequel ledit premier gène thérapeutique est choisi dans le groupe consistant en l'endostatine, l'angiostatine, l'endostatine XVIII, l'endostatine XV, le domaine hémopexine C-terminal de la métalloprotéinase-2 de la matrice, le domaine kringle 5 du plasminogène humain, la monokine induite par l'interféron-gamma (Mig), la protéine 10 inductible par l'interféron-alpha (IP10), le FLT-1 (récepteur de la tyrosine kinase 1 fms-like) soluble et le récepteur du domaine insert kinase (KDR).

2. Vecteur lentiviral de la revendication 1, dans lequel ledit premier gène thérapeutique code pour l'endostatine.

3. Vecteur lentiviral de la revendication 1, dans lequel ledit premier gène thérapeutique code pour l'angiostatine.

4. Vecteur lentiviral de la revendication 1, dans lequel ledit premier gène thérapeutique code pour l'endostatine XVIII.

5. Vecteur lentiviral de la revendication 1, dans lequel ledit premier gène thérapeutique code pour l'endostatine XV.

6. Vecteur lentiviral de la revendication 1, dans lequel ledit premier gène thérapeutique code pour le domaine hémopexine C-terminal de la métalloprotéinase-2 de la matrice.

7. Vecteur lentiviral de la revendication 1, dans lequel ledit premier gène thérapeutique code pour le domaine kringle 5 du plasminogène humain.

8. Vecteur lentiviral de la revendication 1, dans lequel ledit premier gène thérapeutique code pour le Mig.

9. Vecteur lentiviral de la revendication 1, dans lequel ledit premier gène thérapeutique code pour l'IP10.

10. Vecteur lentiviral de la revendication 1, dans lequel ledit premier gène thérapeutique code pour le FLT-1 soluble.

11. Vecteur lentiviral de la revendication 1, dans lequel ledit premier gène thérapeutique code pour le KDR.

12. Vecteur lentiviral de la revendication 1, comprenant en outre un deuxième gène thérapeutique qui inhibe l'angiogenèse, dans lequel ledit deuxième gène thérapeutique est choisi dans le groupe consistant en l'endostatine, l'angiostatine, l'endostatine XVIII, l'endostatine XV, le domaine hémopexine C-terminal de la métalloprotéinase-2 de la matrice, le domaine kringle 5 du plasminogène humain, la monokine induite par l'interféron-gamma (Mig), la protéine 10 inductible par l'interféron-alpha (IP10), le FLT-1 (récepteur de la tyrosine kinase 1 fms-like) soluble et le récepteur du domaine insert kinase (KDR), et dans lequel le vecteur lentiviral code pour une protéine de fusion comprenant une première séquence d'acides aminés codée par le premier gène thérapeutique et une deuxième séquence d'acides aminés codée par le deuxième gène thérapeutique.

13. Vecteur lentiviral de la revendication 1, comprenant en outre un deuxième gène thérapeutique qui inhibe l'angiogenèse, dans lequel ledit deuxième gène thérapeutique est choisi dans le groupe consistant en l'endostatine, l'angiostatine, l'endostatine XVIII, l'endostatine XV, le domaine hémopexine C-terminal de la métalloprotéinase-2 de la matrice, le domaine kringle 5 du plasminogène humain, la monokine induite par l'interféron-gamma (Mig), la protéine 10 inductible par l'interféron-alpha (IP10), le FLT-1 (récepteur de la tyrosine kinase 1 fms-like) soluble et le récepteur du domaine insert kinase (KDR), et dans lequel le vecteur lentiviral comprend un élément IRES (site d'entrée interne des ribosomes) entre ledit premier gène thérapeutique et ledit deuxième gène thérapeutique de telle sorte qu'une première séquence d'acides aminés codée par le premier gène thérapeutique et une deuxième séquence d'acides aminés codée par le deuxième gène thérapeutique soient produites à partir d'un transcrit unique.

14. Vecteur lentiviral de la revendication 12, comprenant en outre un lieur entre la première séquence d'acides aminés et la deuxième séquence d'acides aminés.

15. Vecteur lentiviral de la revendication 12, le vecteur lentiviral codant pour une protéine de fusion de l'endostatine XVIII et de l'angiostatine.

16. Vecteur lentiviral de la revendication 12, le vecteur lentiviral codant pour une protéine de fusion de l'endostatine XVIII et du domaine kringle 5 du plasminogène humain.

17. Vecteur lentiviral de la revendication 12, le vecteur lentiviral codant pour une protéine de fusion du Mig et de l'IP10.

18. Utilisation d'un vecteur lentiviral comprenant un gène thérapeutique qui réduit ou inhibe l'angiogenèse oculaire, pour la fabrication d'un médicament destiné à inhiber la néovascularisation chez un individu ayant une dégénérescence maculaire liée à l'âge, dans laquelle ledit gène thérapeutique est choisi dans le groupe consistant en l'endostatine, l'angiostatine, l'endostatine XVIII, l'endostatine XV, le domaine hémopexine C-terminal de la métalloprotéinase-2 de la matrice, le domaine kringle 5 du plasminogène humain, une protéine de fusion de l'endostatine et de l'angiostatine, une protéine de fusion de l'endostatine et du domaine kringle 5 du plasminogène humain, la monokine induite par l'interféron-gamma (Mig), une protéine 10 inductible par l'interféron-alpha (IP10), une protéine de fusion du Mig et de l'IP10, le FLT-1 (récepteur de la tyrosine kinase 1 fms-like) soluble et le récepteur du domaine insert kinase (KDR).

19. Utilisation selon la revendication 18, dans laquelle le vecteur lentiviral est un vecteur lentiviral tel que présenté dans l'une quelconque des revendications 1-17.

20. Utilisation selon la revendication 18, dans laquelle ledit vecteur lentiviral est administré à une posologie d'environ 10⁶ à 10⁹ particules de transduction dans l'espace capsulaire, vitréen ou sous-rétinien.

21. Procédé de transduction d'un tissu cornéen ex vivo avec un agent thérapeutique, qui comprend l'exposition d'une cellule cornéenne qui fait partie d'un tissu cornéen à un vecteur lentiviral recombinant comprenant ledit gène thérapeutique fonctionnellement couplé à un promoteur connu pour être actif dans les cellules cornéennes, dans lequel le gène thérapeutique est choisi dans le groupe consistant en l'endostatine, l'angiostatine, l'endostatine XVIII, l'endostatine XV, le domaine hémopexine C-terminal de la métalloprotéinase-2 de la matrice, le domaine kringle 5 du plasminogène humain, la monokine induite par l'interféron-gamma (Mig), la protéine 10 inductible par l'interféron-alpha (IP10), le FLT-1 (récepteur de la tyrosine kinase 1 fms-like) soluble et le récepteur du domaine insert kinase (KDR).

22. Tissu cornéen comprenant des cellules transduites avec un vecteur lentiviral recombinant, dans lequel le vecteur lentiviral recombinant comprend un premier gène thérapeutique fonctionnellement couplé à un promoteur connu pour être actif dans des cellules cornéennes, et dans lequel le gène thérapeutique est choisi dans le groupe consistant en l'endostatine, l'angiostatine, l'endostatine XVIII, l'endostatine XV, le domaine hémopexine C-terminal de la métalloprotéinase-2 de la matrice, le domaine kringle 5 du plasminogène humain, la monokine induite par l'interféron-gamma (Mig), la protéine 10 inductible par l'interféron-alpha (IP10), le FLT-1 (récepteur de la tyrosine kinase 1 fms-like) soluble et le récepteur du domaine insert kinase (KDR).

23. Tissu cornéen de la revendication 22, dans lequel les cellules sont transduites avec un vecteur lentiviral tel que présenté dans l'une quelconque des revendications 1-17.

24. Tissu cornéen de la revendication 22, dans lequel le tissu cornéen est un tissu cornéen humain.

25. Tissu cornéen tel que présenté dans l'une quelconque des revendications 22-24 pour utilisation dans le traitement de la néovascularisation cornéenne chez un individu.

26. Tissu cornéen de la revendication 25, le tissu cornéen étant transduit in situ.
